# EUROPEAN PATENT APPLICATION

(11) **EP 4 740 863 A2**
(43) Date of publication of application: **13.05.2026**
(21) Application number: 26169735.3
(22) Date of filing: 27.10.2022
(51) Int. Cl.: A61B 5/372

(54) **SYSTEM AND METHOD FOR DETERMINING A TYPE OF A BRAIN DYSFUNCTION DURING MEDICAL PROCEDURES**

(30) Priority: 14.11.2021 US 202163279132 P; 14.02.2022 US 202263309722 P
(62) Divisional of application: 22892271.2
(71) Applicant: Rambam Med-Tech Ltd., 3109601 Haifa (IL)
(72) Inventor: BAR ON SHAHAF, Dana, 3440210 Haifa (IL); SHAHAF, Goded, 3440210 Haifa (IL)
(74) Representative: Boult Wade Tennant LLP

(57) **Abstract**

A method of determining a type of a brain dysfunction is discloses. The method may include: receiving an electroencephalogram (EEG) signal from an electrode placed on the head; calculating a regularity index for an electric activity of the brain based on EEG signal; detecting a temporal change in the regularity index; receiving a medical related input; and determining the type of brain dysfunction based on the temporal change in the regularity index and the medical related input.

## Description

### CROSS-REFERENCE TO RELATED APPLICATIONS

This application claims the benefit of priority to U.S. Provisional Patent Application Nos. 63/279,132, filed November 14, 2021 entitled "SYSTEM AND METHOD FOR DETERMINING A TYPE OF A BRAIN INJURY DURING A MEDICAL PROCEDURE", and 63/309,722 filed February 14, 2022, entitled "SYSTEM AND METHOD FOR DETERMINING A TYPE OF A BRAIN DYSFUNCTION DURING A MEDICAL PROCEDURE", the contents of which are all incorporated herein by reference in their entirety.

### FIELD OF THE INVENTION

The present invention relates generally to a method of determining a type of brain dysfunction. More specifically, the present invention relates to a system and a method of determining a type of brain dysfunction during a medical procedure or determining the occurrence of an acute fetus brain dysfunction during labor.

### BACKGROUND OF THE INVENTION

Quick identification and classification of brain dysfunction is of the utmost importance. Certain types of dysfunctions, such as stroke, are largely reversible if treated rapidly. While in other conditions, such as delirium, it was shown that early intervention may reduce morbidity and mortality. However, identification and classification of brain dysfunction may be significantly delayed with patients under settings of reduced consciousness and/or reduced communication abilities in medical settings, such as, under anesthesia and in intensive care hospitalization.

Intrapartum fetal monitoring to assess fetal well-being during labor is a key component of intrapartum management. Since certain types of intrapartum fetal distress are largely reversible if treated rapidly, early real time, recognition and identification of intrapartum fetal distress (e.g. due to fetal hypoxia) is one of the greatest challenges of modern obstetrics.

Currently, over 85% of laboring patients undergo intrapartum fetal monitoring by continuous analysis of the fetal heart. Normal components of labor, such as contractions and maternal expulsive efforts, result in transient decreases in gas exchange for the fetus. Even in uncomplicated pregnancies these changes might place the fetus at risk for interruption in oxygenation which might cause fetal intrapartum asphyxia with any associated long-term disabilities. Nevertheless, the efficiency of the current electronic fetal monitoring (EFM) which uses fetal heart characteristics to assess fetal acidemia, hypoxia or fetal brain injury remains poor. A plethora of studies demonstrated that abnormal patterns of the fetal heart rate are of low predictive value for intrapartum fetal hypoxia, metabolic acidosis or brain injury (cerebral palsy CP). According to the Cochrane review, EFM did not decrease the rates of (CP, asphyxia complications or perinatal morbidity and the positive predictive value of non-reassuring FHR patterns for the prediction of CP among singleton newborns with normal birth is only 0.14%

Electroencephalogram (EEG) is a well-established technology for monitoring brain function without the need for the patient's participation. EEG signals can be used to evaluate the degree of similarity of activity over the left and right hemispheres in patients. The degree of similarity or dissimilarity may be indicative of possible brain dysfunction. EEG can further be used for evaluating the degree of regularity of brain activity in patients. The degree of regularity or irregularity may also be indicative of possible brain dysfunction. However, there is no reliable method for identifying the type of brain dysfunction when the patient is under reduced consciousness and/or reduced communication abilities. Therefore, it is of value to generate effective systems to monitor such patients, using EEG, and to identify and classify, in real-time, dysfunction to their brain in order to derive immediate treatment recommendations.

Furthermore, EEG signal can be used to evaluate fetal brain regularity during the process of labor. The degree of regularity, or irregularity, may be indicative of brain dysfunction.

Continuous computerized evaluation of fetal brain regularity using a real-time EEG analysis might improve the sensitivity for early detection of intrapartum fetal brain distress and to derive immediate treatment recommendations.

### SUMMARY OF THE INVENTION

Some aspects of the invention are directed to a method of determining a type of a brain dysfunction, comprising: receiving a first electroencephalogram (EEG) signal from an electrode placed on the first side of head of a patient at a location allowing detecting an electrical activity of a first hemisphere of the brain; receiving a second EEG signal from an electrode placed on the second side of the head of the patient at a location allowing detecting an electrical activity of a second hemisphere of the brain; calculating a similarity index for an electric activity between hemispheres of the brain based on the first and second signals; and detecting a temporal change in the similarity index by determining if the similarity index is at least one of:
(a) below a first threshold value; and
(b) a time derivative of the similarity index is above a second threshold value;

receiving a medical-related input, wherein the medical-related input is at least one of:
   (i) an anesthetic profile during a medical procedure; and
   (ii) an input related to a medical event; and
   determining the type of brain dysfunction based on the temporal change in the similarity index and the medical-related input
receiving the medical-related input includes receiving a signal from at least one of: an external computing device and measurements received from at least one sensor.

In some embodiments, the input related to the medical event measurements is selected from: blood pressure, blood flow, temperature, saturation, glucose levels, other hemodynamic parameters, such as, ECG, central venous pressure and arterial invasive pressure, and target control infusion pumps of anesthetic medications (TCI). In some embodiments, the external computing device is selected from, a controller of a bypass machine, a controller of a medication provision machine, and a controller of the anesthetic machine. In some embodiments, the input related to the medical event is received from one of: blood pressure sensors, thermometer, saturation sensor, US device, X-Ray device, end-tidal CO₂ (EtCO₂) detector, and transcranial Doppler.

In some embodiments, receiving the medical-related input includes receiving an input from a user via a user interface. In some embodiments, the brain dysfunction is postoperative cognitive dysfunction (POCD) and wherein determining a POCD is when the similarity index is below a POCD threshold value under a certain anesthetic profile. In some embodiments, the brain dysfunction is delirium and wherein determining delirium is when: a time derivative of the similarity index is above a delirium threshold value, indicating a decrease in the similarity index with time, and the anesthetic profile shows a temporal reduction in the amount of anesthetic.

In some embodiments, the brain dysfunction is a stroke and wherein determining stroke is when the time derivative of the similarity index is above a first stroke threshold value and the input related to a medical event includes an input related to surgery. In some embodiments, the input related to the surgery is selected from: initiation of cardiopulmonary bypass, weaning from cardiopulmonary bypass received from a controller of a bypass machine. In some embodiments, the input related to the surgery is selected from cannulation or de-cannulation of the aorta, manipulation of the cardiac chambers and valves, and cannulation of the carotid artery for brain perfusion during total circulatory arrest received from a user via a user device.

In some embodiments, the similarity index is calculated based on the amplitudes of the first signal and the second signal. In some embodiments, calculating comprises at least one of, subtraction between the amplitudes and summation of the amplitudes. In some embodiments, the similarity index is calculated as the ratio between the subtraction of the amplitudes and the summation of the amplitudes. In some embodiments, the ratio is calculated for a predetermined period of time.

In some embodiments, calculating the similarity index comprises calculating, over time, at least one of the following using the amplitude of first signal and the amplitude of the second signal: mean difference, median difference, standard deviation, percentile, variance and any combination thereof.

In some embodiments, the similarity index is calculated as differences in power spectrum, between the first signal and the second signal, summarized for one of: specific frequencies, and a frequency band. In some embodiments, calculating the similarity index comprises calculating, over time, at least one of the following using the power spectrum of the first signal and the second signal: mean difference, median difference, standard deviation, percentile, variance and any combination thereof. In some embodiments, calculating the similarity index comprises calculating correlation between powers in the first signal and the second signal over the selected frequencies or the frequency bands. In some embodiments, the correlation is selected from, Pearson correlation and Spearman correlation.

In some embodiments, calculating the similarity index comprises calculating correlation between the powers in the signal and the second signal over the selected periods of time. In some embodiments, the correlation is selected from, Pearson correlation and Spearman correlation.

In some embodiments, the similarity index is calculated based on a degree of shifting in power spectrums distribution over specific frequencies or a band of frequencies between the first signal and the second signal. In some embodiments, the similarity index is calculated based on a degree of shifting in power spectrums distribution over time between the first signal and the second signal.

Some additional aspects of the invention may be related to a method of determining a brain dysfunction, comprising:
receiving an electroencephalogram (EEG) signal from an electrode placed on a patient's head at a location allowing detecting electrical activity of the brain;
calculating regularity index for the electric activity of the brain based on the EEG signal; and
detecting a regularity change in the regularity index by determining if the regularity index is at least one of:
   (a) below a first threshold value; and
   (b) a time derivative of the regularity index is above a second threshold value;
receiving a medical-related input, wherein the medical-related input is at least one of:
   (i) input related to the patient's medical history;
   (ii) an anesthetic profile during a medical procedure; and
   (iii) an input related to a medical event; and
determining a type of brain dysfunction based on the temporal change in the regularity index and the medical-related input.

In some embodiments, receiving input related to the patient related to the patient's medical history comprising at least one of: an age of the patient, a baseline cognitive profile and cardiovascular risk assessment.

In some embodiments, receiving the medical related input includes receiving a signal from at least one of: an external computing device and measurements received form at least one sensor. In some embodiments, the input related to the medical event measurements is selected from: blood pressure, blood flow, temperature, saturation, glucose levels, other hemodynamic parameters, such as, pre-operative demographic and/or clinical data, ECG, central venous pressure, and arterial invasive pressure, patient movements, target control infusion pumps of anesthetic medications (TCI) and patient movements monitoring. In some embodiments, the external computing device is selected from a data storage with an electronic medical record, a controller of a bypass machine, a controller of a medication provision machine, a motion monitoring device, and a controller of the anesthetic machine.

In some embodiments, the input related to the medical event is received from one of: electronic medical records, blood pressure sensors, thermometer, camera, saturation sensor, US device, X-Ray device, end-tidal CO₂ (EtCO2) detector, motion detector, and transcranial Doppler.

In some embodiment, wherein receiving the input related to the medical event includes receiving input from a user via a user interface.

In some embodiments, the brain dysfunction is perioperative neurocognitive dysfunction (PND). In some embodiment, the PND includes preoperatively diagnosed cognitive decline, postoperative delirium, delayed neurocognitive recovery, and postoperative cognitive dysfunction (POCD). In some embodiment, determining a POCD is when the regularity index is below a POCD threshold value under certain anesthetic profile.

In some embodiments, the brain dysfunction is delirium and wherein determining delirium is when: a time derivative of the regularity index is above a delirium threshold value, indicating a decrease in the regularity index with time, and the anesthetic profile shows temporal reduction in the amount of anesthetic.

In some embodiments, the brain dysfunction is a stroke and wherein determining stroke is when the time derivative of the regularity index is above a first stroke threshold value and the input related to a medical event includes an input related to a surgery.

In some embodiments, the input related to the surgery is selected from: weaning to or from cardiopulmonary bypass received from a controller of a bypass machine. In some embodiment, the input related to the surgery is selected from cannulation or de-cannulation of the aorta, manipulation of the cardiac chambers and valves, cannulation of the carotid artery for brain perfusion during total circulatory arrest, bypass duration, etc. received from a user via a user device.

In some embodiments, the brain dysfunction is a seizure and wherein determining seizure is when: a time derivative of the regularity index is above a seizure threshold value, indicating a decrease in the regularity index with time, followed by a time derivative of the regularity index above another seizure threshold value. In some embodiments, the input related to a medical event include an input related to surgery or hospitalization. In some embodiments, the input related to the surgery is aberrant patient movements, detected by one or more motion sensors.

In some embodiments, the regularity index is calculated based on the amplitude of the signal. In some embodiment, calculating comprises evaluating variance among the wave amplitudes.

In some embodiments, the regularity index is calculated based on the change between consecutive wave amplitudes. In some embodiments, the index is calculated for a predetermined period of time.

In some embodiments, calculating the regularity index comprises calculating, over time, at least one of the following using the amplitude of the signal: mean variability, median variability, standard deviation, percentile, variance, and any combination thereof.

In some embodiments, the regularity index is calculated based on the power spectrum, of the signal, summarized for one of: specific frequencies, and a frequency band.

In some embodiments, calculating the regularity index comprises calculating, over time, at least one of the following using the power spectrum of the signal: mean regularity, median regularity, standard deviation, percentile, variance, and any combination thereof.

In some embodiments, calculating the regularity index comprises calculating powers in the signal over the selected frequencies or the frequency bands, which are associated with decreased or increased regularity.

In some embodiments, calculating the regularity index comprises calculating the powers in the signal over the selected periods of time.

In some embodiments, the regularity index is calculated based on a degree of shifting in power spectrums distribution over specific frequencies or a band of frequencies in the signal.

Some additional aspects of the invention may be related to a method of determining fetal brain dysfunction during labor, comprising:
(a) receiving an electroencephalogram (EEG) signal from a single channel electrode placed on the head of the fetus during delivery;
(b) calculating a regularity index for the electric activity;
(c) detecting a temporal change in the regularity index by determining if the regularity index is at least one of:
   (i) below a first threshold value; and/or
   (ii) a time derivative of the regularity index is above a second threshold value; and
(d) determining brain dysfunction of the fetus based on the temporal change in the regularity index.

In some embodiments, the method further comprises receiving a medical related input; and determining the brain dysfunction of the fetus also based on the medical related input. In some embodiments, receiving the medical related input includes receiving a signal from at least one of: an external computing device and measurements received form at least one sensor. In some embodiments, the input related to the medical event measurements is selected from: fetus ECG changes, uterine contractions, and the clinical condition of the mother such as hypo or hypertension, hemodynamic status, hypoxia, hypoglycemia, pre-eclampsia and eclampsia status and seizures. In some embodiments, the external computing device is selected from a controller of fetus ECG, and a controller of a tocodynamometer.

In some embodiments, receiving the input related to the medical event includes receiving an input from a user via a user interface.

In some embodiments, the regularity index is calculated based on the amplitudes of the signal. In some embodiments, calculating comprises at least one of differences between amplitudes of consecutive waves and summation of the differences. In some embodiments, the regularity index is calculated as the ratio between the count of the amplitude differences below a threshold to the total of consecutive peak pairs. In some embodiments, the ratio is calculated for a predetermined period of time. In some embodiments, calculating the regularity index comprises calculating, over time, at least one of the following using the amplitude of signal: mean, median, standard deviation, percentile, variance and any combination thereof.

In some embodiments, the regularity index is calculated from the power spectrum, summarized for one of: (i) specific frequencies, and (ii) a frequency band. In some embodiments, calculating the regularity index comprises calculating, over time, at least one of the following using the power spectrum of the signal: mean, median, standard deviation, percentile, variance and any combination thereof. In some embodiments, calculating the regularity index comprises calculating the power of the signal over the selected frequencies or the frequency bands.

Further aspects of the present disclosure are set forth in the following numbered clauses:-
Clause 1 A method of determining a type of a brain dysfunction, comprising:
   receiving a first electroencephalogram (EEG) signal from an electrode placed on the first side of head of a patient at a location allowing detecting an electrical activity of a first hemisphere of the brain;
   receiving a second EEG signal from an electrode placed on the second side of the head of the patient at a location allowing detecting an electrical activity of a second hemisphere of the brain;
   calculating a similarity index for an electric activity between hemispheres of the brain based on the first and second signals; and
   detecting a temporal change in the similarity index by determining if the similarity index is at least one of:
      (a) below a first threshold value; and
      (b) a time derivative of the similarity index is above a second threshold value;

      receiving a medical related input, wherein the medical related input is at least one of:
         (i) an anesthetic profile during a medical procedure; and
         (ii) an input related to a medical event; and
      determining the type of brain dysfunction based on the temporal change in the similarity index and the medical related input.
Clause 2 The method of clause 1, wherein receiving the medical related input includes receiving a signal from at least one of: an external computing device and measurements received form at least one sensor.
Clause 3 The method of clause 2, wherein the input related to the medical event measurements is selected from: blood pressure, blood flow, temperature, saturation, glucose levels, other hemodynamic parameters, such as, ECG, central venous pressure and arterial invasive pressure, and target control infusion pumps of anesthetic medications (TCI).
Clause 4 The method of clause 2, wherein the external computing device is selected from, a controller of a bypass machine, a controller of a medication provision machine, and a controller of the anesthetic machine.
Clause 5 The method according to any one of clauses 1 to 4, wherein the input related to the medical event is received from one of: blood pressure sensors, thermometer, saturation sensor, US device, X-Ray device, end-tidal CO₂ (EtCO₂) detector, and trans cranial Doppler.
Clause 6 The method according to any one of clauses 1 to 5, wherein receiving the input related to the medical event includes receiving an input from a user via a user interface.
Clause 7 The method according to any one of clauses 1 to 6, wherein the brain dysfunction is post operative cognitive dysfunction (POCD) and wherein determining a POCD is when the similarity index is below a POCD threshold value under certain anesthetic profile.
Clause 8 The method according to any one of clauses 1 to 6, wherein the brain dysfunction is delirium and wherein determining delirium is when: a time derivative of the similarity index is above a delirium threshold value, indicating a decrease in the similarity index with time, and the anesthetic profile shows temporal reduction in the amount of anesthetic.
Clause 9 The method according to any one of clauses 1 to 6, wherein the brain dysfunction is a stroke and wherein determining stroke is when the time derivative of the similarity index is above a first stroke threshold value and the input related to a medical event includes an input related to a surgery.
Clause 10 The method of clause 9, wherein the input related to the surgery is selected from: initiation of cardiopulmonary bypass and weaning from cardiopulmonary bypass received from a controller of a bypass machine.
Clause 11 The method of clause 9, wherein the input related to the surgery is selected from: cannulation or de-cannulation of aorta, manipulation of the cardiac chambers and valves and cannulation of carotid artery for brain perfusion during total circulatory arrest received from a user via a user device.
Clause 12 The method according to any one of clauses 1 to 11, wherein the similarity index is calculated based on the amplitudes of the first signal and the second signal.
Clause 13 The method of clause 12, where calculating comprises at least one of, subtraction between of the amplitudes and summation of the amplitudes.
Clause 14 The method of clause 13, wherein the similarity index is calculated as the ratio between the subtraction of the amplitudes and the summation of the amplitudes.
Clause 15 The method of clause 14, wherein the ratio is calculated for a predetermined period of time.
Clause 16 The method of clause 12, wherein calculating the similarity index comprises calculating, over time, at least one of the following using the amplitude of first signal and the amplitude of the second signal: mean difference, median difference, standard deviation, percentile, variance and any combination thereof.
Clause 17 The method according to any one of clauses 1 to 11, wherein the similarity index is calculated as differences in power spectrum, between the first signal and the second signal, summarized for one of: specific frequencies, and a frequency band.
Clause 18 The method of clause 17, wherein calculating the similarity index comprises calculating, over time, at least one of the following using the power spectrum of the first signal and the second signal: mean difference, median difference, standard deviation, percentile, variance and any combination thereof.
Clause 19 The method of clause 17, wherein calculating the similarity index comprises calculating correlation between powers in the first signal and the second signal over the selected frequencies or the frequency bands.
Clause 20 The method of clause 19, wherein the correlation is selected from, Pearson correlation and Spearman correlation.
Clause 21 The method according to any one of clauses 1 to 11, wherein calculating the similarity index comprises calculating correlation between the powers in the signal and the second signal over the selected periods of time.
Clause 22 The method of clause 21, wherein the correlation is selected from, Pearson correlation and Spearman correlation.
Clause 23 The method according to any one of clauses 1 to 11, wherein the similarity index is calculated based on a degree of shifting in power spectrums distribution over specific frequencies or a band of frequencies between the first signal and the second signal.
Clause 24 The method according to any one of clauses 1 to 11, wherein the similarity index is calculated based on a degree of shifting in power spectrums distribution over time between the first signal and the second signal.
Clause 25 A method of determining a brain dysfunction, comprising:
   receiving an electroencephalogram (EEG) signal from an electrode placed on a patient' head at a location allowing detecting electrical activity of the brain;
   calculating regularity index for the electric activity of the brain based on the EEG signal; and
   detecting a regularity change in the regularity index by determining if the regularity index is at least one of:
      (a) below a first threshold value; and
      (b) a time derivative of the regularity index is above a second threshold value;
   receiving a medical related input, wherein the medical related input is at least one of:
      (i) input related to the patient's medical history;
      (ii) an anesthetic profile during a medical procedure; and
      (iii) an input related to a medical event; and
   determining a type of brain dysfunction based on the temporal change in the regularity index and the medical related input.
Clause 26 The method of clause 25, wherein the receiving input related to the patient related to the patient's medical history comprising at least one of: an age of the patient, a baseline cognitive profile and cardiovascular risk assessment.
Clause 27 The method according to any one of clauses 25 to 26, wherein receiving the medical related input includes receiving a signal from at least one of: an external computing device and measurements received form at least one sensor.
Clause 28 The method of clause 27, wherein the input related to the medical event measurements is selected from: blood pressure, blood flow, temperature, saturation, glucose levels, other hemodynamic parameters, such as, pre-operative demographic and/or clinical data, ECG, central venous pressure and arterial invasive pressure, patient movements, target control infusion pumps of anesthetic medications (TCI) and patient movements monitoring.
Clause 29 The method of clause 27, wherein the external computing device is selected from a data storage with an electronic medical record, a controller of a bypass machine, a controller of a medication provision machine, a motion monitoring device, and a controller of the anesthetic machine.
Clause 30 The method according to any one of clauses 27 to 29, wherein the input related to the medical event is received from one of: electronic medical record, blood pressure sensors, thermometer, camera, saturation sensor, US device, X-Ray device, end-tidal CO₂ (EtCO2) detector, motion detector, and transcranial Doppler.
Clause 31 The method according to any one of clauses 28 to 30, wherein receiving the input related to the medical event includes receiving an input from a user via a user interface.
Clause 32 The method according to any one of clauses 28 to 30, wherein the brain dysfunction is perioperative neurocognitive dysfunction (PND).
Clause 33 The method of clause 32, wherein the PND includes preoperatively diagnosed cognitive decline, postoperative delirium, delayed neurocognitive recovery, and postoperative cognitive dysfunction (POCD).
Clause 34 The method of clause 33, wherein determining a POCD is when the regularity index is below a POCD threshold value under certain anesthetic profile.
Clause 35 The method according to any one of clauses 28 to 30, wherein the brain dysfunction is delirium and wherein determining delirium is when: a time derivative of the regularity index is above a delirium threshold value, indicating a decrease in the regularity index with time, and the anesthetic profile shows temporal reduction in the amount of anesthetic.
Clause 36 The method according to any one of clauses 28 to 30, wherein the brain dysfunction is a stroke and wherein determining stroke is when the time derivative of the regularity index is above a first stroke threshold value and the input related to a medical event includes an input related to a surgery.
Clause 37 The method of clause 36, wherein the input related to the surgery is selected from: weaning to or from cardiopulmonary bypass received from a controller of a bypass machine.
Clause 38 The method of clause 36, wherein the input related to the surgery is selected from cannulation or de-cannulation of the aorta, manipulation of the cardiac chambers and valves, cannulation of the carotid artery for brain perfusion during total circulatory arrest, bypass duration, etc. received from a user via a user device.
Clause 39 The method according to any one of clauses 28 to 30, wherein the brain dysfunction is a seizure and wherein determining seizure is when: a time derivative of the regularity index is above a seizure threshold value, indicating a decrease in the regularity index with time, followed by a time derivative of the regularity index above another seizure threshold value.
Clause 40 The method of clause 39, wherein the input related to a medical event include an input related to a surgery or hospitalization.
Clause 41 The method of clause 39, wherein the input related to the surgery is aberrant patient movements, detected by one or more motion sensors.
Clause 42 The method according to any one of clauses 25 to 41, wherein the regularity index is calculated based on the amplitude of the signal.
Clause 43 The method of clause 42, wherein calculating comprises evaluating variance among the wave amplitudes.
Clause 44 The method according to any one of clauses 25 to 41, wherein the regularity index is calculated based on the change between consecutive wave amplitudes. In some embodiments, the index is calculated for a predetermined period of time.
Clause 45 The method according to any one of clauses 25 to 41, wherein calculating the regularity index comprises calculating, over time, at least one of the following using the amplitude of the signal: mean variability, median variability, standard deviation, percentile, variance and any combination thereof.
Clause 46 The method according to any one of clauses 25 to 41, wherein the regularity index is calculated based on the power spectrum, of the signal, summarized for one of: specific frequencies, and a frequency band.
Clause 47 The method according to any one of clauses 25 to 41, wherein calculating the regularity index comprises calculating, over time, at least one of the following using the power spectrum of the signal: mean regularity, median regularity, standard deviation, percentile, variance and any combination thereof.
Clause 48 The method according to any one of clauses 25 to 41, wherein calculating the regularity index comprises calculating powers in the signal over the selected frequencies or the frequency bands, which associate with decreased or increased regularity.
Clause 49 The method according to any one of clauses 25 to 41, wherein calculating the regularity index comprises calculating the powers in the signal over the selected periods of time.
Clause 50 The method according to any one of clauses 25 to 41, wherein regularity index is calculated based on a degree of shifting in power spectrums distribution over specific frequencies or a band of frequencies in the signal.
Clause 51 A method for determining brain dysfunction of a fetus during delivery, comprising:
   (a) receiving an electroencephalogram (EEG) signal from a single channel electrode placed on the head of the fetus during delivery;
   (b) calculating a regularity index for the electric activity;
   (c) detecting a temporal change in the regularity index by determining if the regularity index is at least one of:
      (i) below a first threshold value; and/or
      (ii) a time derivative of the regularity index is above a second threshold value; and
   (d) determining brain dysfunction of the fetus based on the temporal change in the regularity index.
Clause 52 The method of clause 51, further comprising:
   receiving a medical related input; and
   determining the brain dysfunction of the fetus also based on the medical related input.
Clause 53 The method of clause 52, wherein receiving the medical related input includes receiving a signal from at least one of: an external computing device and measurements received form at least one sensor.
Clause 54 The method of clause 53, wherein the input related to the medical event measurements is selected from: fetus ECG changes, uterine contractions, and the clinical condition of the mother such as hypo or hypertension, hypoxia, hypoglycemia, pre-eclampsia and eclampsia status and seizures.
Clause 55 The method of clause 54, wherein the external computing device is selected from a controller of fetus ECG, and a controller of a tocodynamometer.
Clause 56 The method according to any one of clause 50 to 55, wherein receiving the input related to the medical event includes receiving an input from a user via a user interface.
Clause 57 The method according to any one of clauses 50 to 56, wherein the regularity index is calculated based on the amplitudes of the signal.
Clause 58 The method of clause 57, where calculating comprises at least one of differences between amplitudes of consecutive waves and summation of the differences.
Clause 59 The method of clause 58, wherein the regularity index is calculated as the ratio between the count of the amplitude differences below a threshold to the total of consecutive peak pairs.
Clause 60 The method of clause 59, wherein the ratio is calculated for a predetermined period of time.
Clause 61 The method of clause 60, wherein calculating the regularity index comprises calculating, over time, at least one of the following using the amplitude of signal: mean, median, standard deviation, percentile, variance and any combination thereof.
Clause 62 The method according to any one of clauses 50 to 61, wherein the regularity index is calculated from the power spectrum, summarized for one of: (i) Specific frequencies, and (ii) A frequency band.
Clause 63 The method of clause 62, wherein calculating the regularity index comprises calculating, over time, at least one of the following using the power spectrum of the signal: mean, median, standard deviation, percentile, variance and any combination thereof.
Clause 64 The method of clause 63, wherein calculating the regularity index comprises calculating the power of the signal over the selected frequencies or the frequency bands.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:
Fig. 1A is an illustration of the positioning of EEG electrodes on two hemispheres of a patient's head according to some embodiments of the invention;
Fig. 1B is a block diagram of a system for determining a type of brain dysfunction using readings from two hemispheres of a patient's brain, according to some embodiments of the invention;
Fig. 1C is an illustration of the positioning of EEG electrodes on a patient's head according to some embodiments of the invention;
Fig. 1D is a block diagram of a system for determining a type of brain dysfunction, according to some embodiments of the invention;
Fig. 1E is an illustration of the positioning of EEG electrodes on a fetus head and also possibly on the mother according to some embodiments of the invention;
Fig. 1F is a block diagram, depicting a computing device which may be included in a system for determining brain dysfunction, according to some embodiments of the invention;
Fig. 2 is a flowchart of a method of determining a type of a brain dysfunction, according to some embodiments of the invention;
Fig. 3 is an illustration of an EEG plot from two hemispheres of a patient's brain according to some embodiments of the invention;
Fig. 4 shows graphs of lateral interconnection ratio (LIR) calculated for several patients showing several types of dysfunctions in comparison with a normal brain function, according to some embodiments of the invention;
Fig. 5 shows graphs of accumulated LIR data from groups of patients having several types of dysfunctions in comparison with patients having a normal brain function, according to some embodiments of the invention;
Fig. 6 is a flowchart of another method of determining a type of a brain dysfunction, according to some embodiments of the invention;
Fig. 7 is an illustration of an EEG plot of a patient's brain according to some embodiments of the invention;
Figs. 8A-8B show graphs of regularity index calculated for several patients showing stroke and seizure, in comparison with a normal brain, according to some embodiments of the invention;
Figs. 9A-9B show graphs of the regularity index calculated for several patients according to some embodiments of the invention;
Fig. 10 is a flowchart of a method of determining an acute brain dysfunction, according to some embodiments of the invention;
Fig. 11 is an illustration of an EEG plot according to some embodiments of the invention; and
Figs. 12, 12A, 12B, 12C, and 12D are graphs of regularity calculated for two levels of asphyxia, in comparison with pregnancy-related anomalies and normal brain during delivery, according to some embodiments of the invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE PRESENT INVENTION

One skilled in the art will realize the invention may be embodied in other specific forms without departing from the spirit or essential characteristics thereof. The foregoing embodiments are therefore to be considered in all respects illustrative rather than limiting of the invention described herein. Scope of the invention is thus indicated by the appended claims, rather than by the foregoing description, and all changes that come within the meaning and range of equivalency of the claims are therefore intended to be embraced therein.

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention. Some features or elements described with respect to one embodiment may be combined with features or elements described with respect to other embodiments. For the sake of clarity, discussion of same or similar features or elements may not be repeated.

Although embodiments of the invention are not limited in this regard, discussions utilizing terms such as, for example, "processing," "computing," "calculating," "determining," "establishing", "analyzing", "checking", or the like, may refer to operation(s) and/or process(es) of a computer, a computing platform, a computing system, or other electronic computing device, that manipulates and/or transforms data represented as physical (e.g., electronic) quantities within the computer's registers and/or memories into other data similarly represented as physical quantities within the computer's registers and/or memories or other information non-transitory storage medium that may store instructions to perform operations and/or processes.

Although embodiments of the invention are not limited in this regard, the terms "plurality" and "a plurality" as used herein may include, for example, "multiple" or "two or more". The terms "plurality" or "a plurality" may be used throughout the specification to describe two or more components, devices, elements, units, parameters, or the like. The term "set" when used herein may include one or more items.

Unless explicitly stated, the method embodiments described herein are not constrained to a particular order or sequence. Additionally, some of the described method embodiments or elements thereof can occur or be performed simultaneously, at the same point in time, or concurrently.

Embodiments of the present invention disclose a method and a system for determining a type of brain dysfunction during a surgery, an emergency room, in an intensive care unit, or other medical procedure when the patient has reduced consciousness and/or reduced communication ability. The method may include receiving two EEG signals from EEG electrodes located at two sides of the brain and calculating a temporal similarity index indicating the dissimilarity in activity between the two hemispheres of the brain. Additionally, the method may include receiving a medical-related input in real-time (e.g., timing events in the operating room or in the intensive care unit), which when combined with the temporal similarity index may diagnose the distinct type of brain dysfunction and may generate appropriate treatment recommendations.

Additional embodiments of the present invention disclose a method and a system for determining a type of brain dysfunction during a surgery, an emergency room, in an intensive care unit, or other medical setup or procedure when the patient has reduced consciousness and/or reduced communication ability. The reduced consciousness may occur during anesthesia, sleep, or after brain injury, or systemic body disorder, which affects the brain. The method may include receiving at least a single EEG signal from a single EEG channel and calculating a temporal regularity index indicating the irregularity in the activity of the brain. Additionally, the method may include receiving a medical-related input in real-time (e.g., timing events in the operating room or in the intensive care unit) or in advance (e.g. patient age, and risk factors), which when combined with the temporal regularity index may diagnose the distinct type of brain dysfunction and may generate appropriate treatment recommendations.

Additional embodiments of the present invention disclose a method and a system for determining the occurrence of acute brain dysfunction during fetus delivery. The method may include receiving at least a single EEG signal from at least a single EEG channel and calculating a temporal regularity index indicating the irregularity in activity of the brain. Additionally, the method may include receiving a medical related input in real time (e.g., timing events in the operating room or in the intensive care unit), or in advance (e.g. patient age, and risk factors), which when combined with the temporal regularity index may diagnose the occurrence of acute brain dysfunction and may generate appropriate treatment recommendations.

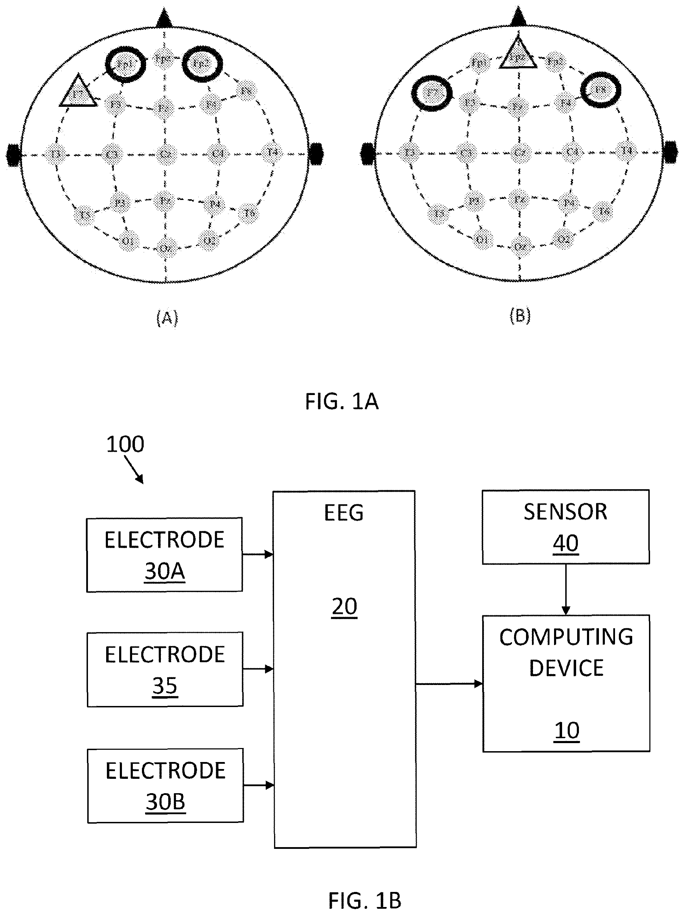

Reference is now made to Fig. 1A which includes illustrations of two optional positions of EEG electrodes over a patient's head. The two nonlimiting positions enable the placement of the electrodes below the hairline. EEG is measured by sampling the potential differences between a target electrode and a reference electrode. In the nonlimiting example illustrated in Fig. 1A , the target electrodes are circled, and the reference electrode is marked with a triangle. Therefore, in the two nonlimiting positions illustrated in Fig. 1A , there are two channels (left and right) with a common reference.

In some embodiments, the first target electrode is placed on the first side (e.g., left) of the head midline, and a second target electrode is placed on a second side (e.g., right) of the head midline. The reference electrode(s) could be placed freely and could be the same for the two or more target electrodes, or could otherwise be different, but in approximate symmetrical positions on the axis between the first and second sides. In some embodiments, all electrodes could be placed below the hairline.

Reference is now made to Fig. 1B which is a block diagram of a system for determining a type of brain dysfunction, according to some embodiments of the invention. As used herein, brain dysfunction is characterized by an inability to pay attention, disorientation, an inability to think clearly, a complete loss of consciousness, to impairment of one or several of the many specific functions that contribute to conscious experience, or to motor, sensory or cognitive function. The type and severity of brain dysfunction depend on how extensive brain damage is and where it is located. There are several types of brain dysfunctions, for example, stroke, seizure, postoperative cognitive dysfunction (POCD), delirium, and the like.

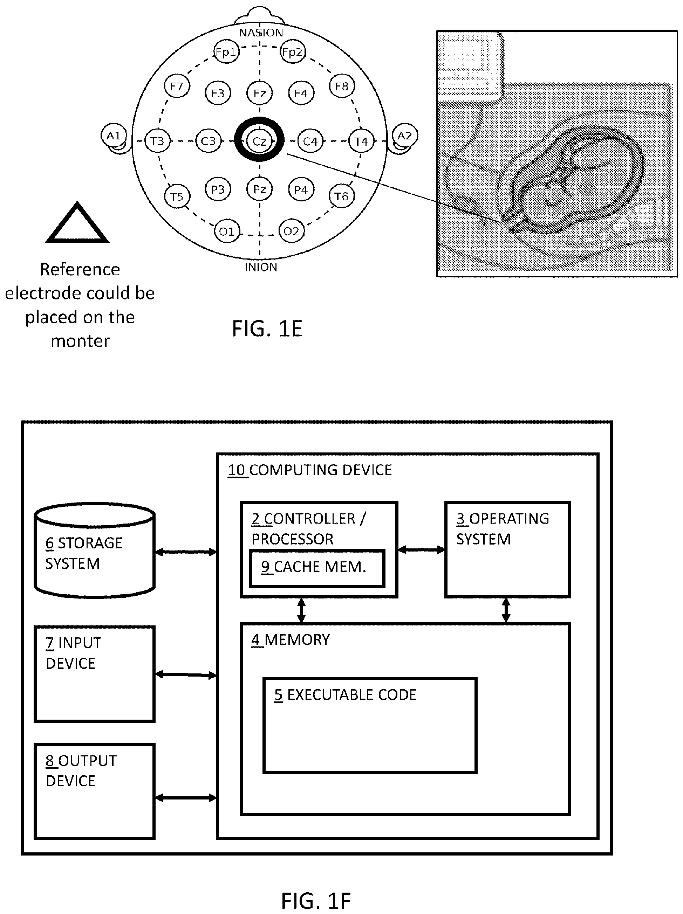

A system 100 may include a computing device 10, illustrated and discussed with respect to Fig. 1E, an EEG system 20 for measuring EEG signals from at least two target electrodes 30A and 30B and at least one reference electrode 35. EEG system 20 may be any EEG system/device known in the art. Computing device 10 may be configured to receive signals from two or more target electrodes 30A and 30B, additional sensors 40 associated with the patient and/or the intensive care unit (ICU)/operation/emergency/room and from external computing and/or user devices, for example, via the internet, wired and/or wireless communication. In some embodiments, one or more sensors 40 may be selected from blood pressure sensors, thermometer, saturation sensor, Ultrasound (US) device, X-Ray device, end-tidal CO₂ (EtCO₂) detector, transcranial Doppler, and the like. Other signals may be received from, cardiopulmonary bypass (such as, mean arterial pressure on pump versus regular biphasic pressure), input from extracorporeal membrane oxygenation (ECMO) device, arterial blood gas, central venous pressure, level of anesthesia (such as, minimal alveolar concentration (MAC)), a dose of IV anesthetics which may be taken from a device such as a target control infusion (TCI) pump or other automatic anesthetic infusion pumps, and the like.

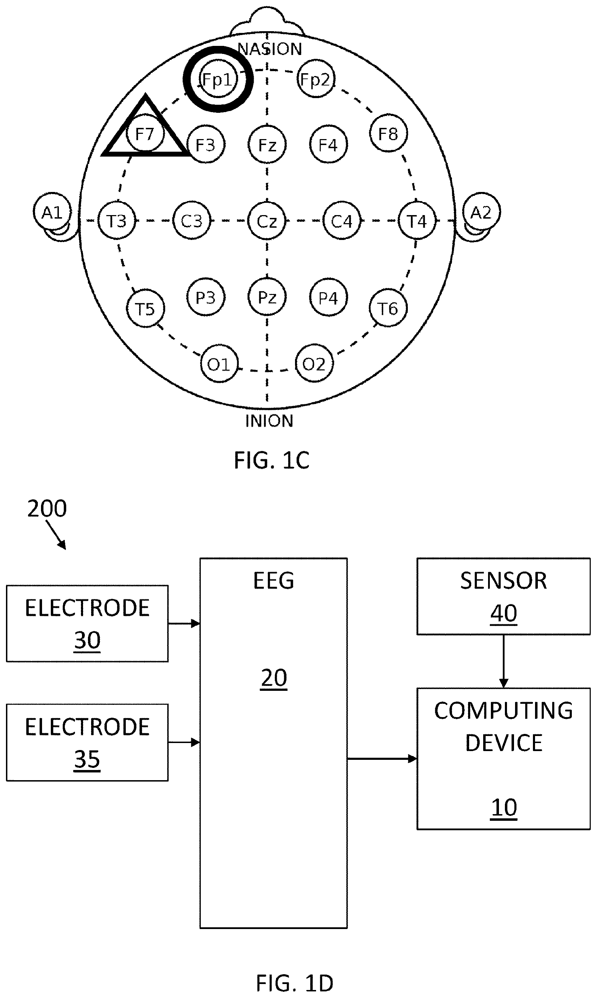

Reference is now made to Fig. 1C which includes illustrations of an optional position of EEG electrodes over a patient's head. The nonlimiting position enables the placement of the electrodes below the hairline. EEG is measured by sampling the potential differences between a target electrode and a reference electrode. In the nonlimiting example illustrated in Fig. 1C, the single target electrode is circled, and the reference electrode is marked with a triangle. Therefore, in the nonlimiting position illustrated in Fig. 1C, there is a single channel with a reference.

In some embodiments, the target electrode is placed near the head midline. The reference electrode(s) could be placed freely. In some embodiments, all electrodes could be placed below the hairline.

Reference is now made to Fig. 1D which is a block diagram of a system for determining a type of brain dysfunction, according to some embodiments of the invention. The system of Fig. 1D may also refer to a system for determining an acute brain dysfunction during delivery, according to some embodiments of the invention.

A system 200 may include a computing device 10, illustrated and discussed with respect to Fig. 1E, an EEG system 20 for measuring EEG signals from at least one target electrode 30 and at least one reference electrode 35. EEG system 20 may be any EEG system/device known in the art. Computing device 10 may be configured to receive signals from one or more target channels 30, additional sensors 40 associated with the patient and/or the intensive care unit (ICU)/pediatric ICU (PICU)/ Neonatal ICU (NICU)/operation/emergency/room and from external computing and/or user devices, for example, via the internet, wired and/or wireless communication. Alternatively, computing device 10 may be configured to receive signals from one or more target channels 30, additional sensors 40 associated with the fetus and the mother and from external computing and/or user devices, for example, via the internet, wired and/or wireless communication.

In some embodiments, one or more sensors 40 may be selected from blood pressure sensors, thermometer, saturation sensor, Ultrasound (US) device, X-Ray device, end-tidal CO₂ (EtCO₂) detector, transcranial Doppler, and the like. Other signals may be received from, cardiopulmonary bypass (such as, mean arterial pressure on pump versus regular biphasic pressure), input from extracorporeal membrane oxygenation (ECMO) device, arterial blood gas, central venous pressure, level of anesthesia (such as, minimal alveolar concentration (MAC)), a dose of IV anesthetics which may be taken from a device such as a target control infusion (TCI) pump or other automatic anesthetic infusion pumps, movement detectors and the like.

Reference is now made to Fig. 1E which includes illustrations of an optional position of EEG electrode over a fetus head. The nonlimiting position enables the placement of the electrode with various head presentations during delivery. EEG is measured by sampling the potential differences between a target electrode and a reference electrode. In the nonlimiting example illustrated in Fig. 1E, the target electrode is circled, and the reference electrode is marked with a triangle. This reference electrode could be positioned comfortably on the mother. Therefore, in the nonlimiting position illustrated in Fig. 1E, there is a channel with a reference.

Reference is now made to Fig. 1F, which is a block diagram depicting a computing device, which may be included within an embodiment of a system for determining a type of brain dysfunction during a surgery, according to some embodiments.

Computing device 10 may include a processor or controller 2 that may be, for example, a central processing unit (CPU) processor, a chip or any suitable computing or computational device, an operating system 3, a memory 4, executable code 5, a storage system 6, input devices 7 and output devices 8. Processor 2 (or one or more controllers or processors, possibly across multiple units or devices) may be configured to carry out methods described herein, and/or to execute or act as the various modules, units, etc. More than one computing device 10 may be included in, and one or more computing devices 10 may act as the components of, a system according to embodiments of the invention.

Operating system 3 may be or may include any code segment (e.g., one similar to executable code 5 described herein) designed and/or configured to perform tasks involving coordination, scheduling, arbitration, supervising, controlling, or otherwise managing operation of computing device 10, for example, scheduling execution of software programs or tasks or enabling software programs or other modules or units to communicate. Operating system 3 may be a commercial operating system. It will be noted that an operating system 3 may be an optional component, e.g., in some embodiments, a system may include a computing device that does not require or include an operating system 3.

Memory 4 may be or may include, for example, a Random Access Memory (RAM), a read only memory (ROM), a Dynamic RAM (DRAM), a Synchronous DRAM (SD-RAM), a double data rate (DDR) memory chip, a Flash memory, a volatile memory, a nonvolatile memory, a cache memory, a buffer, a short term memory unit, a long term memory unit, or other suitable memory units or storage units. Memory 4 may be or may include a plurality of possibly different memory units. Memory 4 may be a computer or processor non-transitory readable medium, or a computer non-transitory storage medium, e.g., a RAM. In one embodiment, a non-transitory storage medium such as memory 4, a hard disk drive, another storage device, etc. may store instructions or code which when executed by a processor may cause the processor to carry out methods as described herein.

Executable code 5 may be any executable code, e.g., an application, a program, a process, task or script. Executable code 5 may be executed by processor or controller 2 possibly under control of operating system 3. For example, executable code 5 may be an application that may determine a type of a brain dysfunction during a surgery as further described herein. Although, for the sake of clarity, a single item of executable code 5 is shown in Fig. 1C, a system according to some embodiments of the invention may include a plurality of executable code segments similar to executable code 5 that may be loaded into memory 4 and cause processor 2 to carry out methods described herein.

Storage system 6 may be or may include, for example, a flash memory as known in the art, a memory that is internal to, or embedded in, a micro controller or chip as known in the art, a hard disk drive, a CD-Recordable (CD-R) drive, a Blu-ray disk (BD), a universal serial bus (USB) device or other suitable removable and/or fixed storage unit. Data related to EEG signals in storage system 6 and may be loaded from storage system 6 into memory 4 where it may be processed by processor or controller 2. In some embodiments, some of the components shown in Fig. 1C may be omitted. For example, memory 4 may be a nonvolatile memory having the storage capacity of storage system 6. Accordingly, although shown as a separate component, storage system 6 may be embedded or included in memory 4.

Input devices 7 may be or may include any suitable input devices, components or systems, e.g., a detachable keyboard or keypad, a mouse and the like. Output devices 8 may include one or more (possibly detachable) displays or monitors, speakers and/or any other suitable output devices. Any applicable input/output (I/O) devices may be connected to Computing device 10 as shown by blocks 7 and 8. For example, a wired or wireless network interface card (NIC), a universal serial bus (USB) device or external hard drive may be included in input devices 7 and/or output devices 8. It will be recognized that any suitable number of input devices 7 and output device 8 may be operatively connected to Computing device 10 as shown by blocks 7 and 8.

A system according to some embodiments of the invention may include components such as, but not limited to, a plurality of central processing units (CPU) or any other suitable multi-purpose or specific processors or controllers (e.g., similar to element 2), a plurality of input units, a plurality of output units, a plurality of memory units, and a plurality of storage units.

Systems 100 and/or 200 may include a computing device such as computing device 10 of Fig. 1E and may be adapted to execute one or more modules of executable code (e.g., element 5 of Fig. 1E) to determine a type of a brain dysfunction, as further described herein.

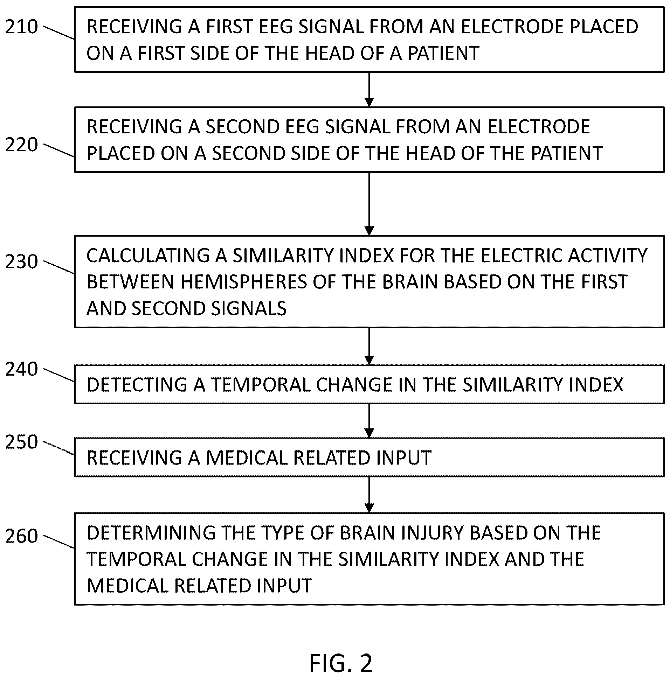

Reference is now made to Fig. 2, which is a flowchart of a method of determining a type of a brain dysfunction according to some embodiments of the invention. The method of Fig. 2 may be executed by any computing device, for example, computing device 10 based on a code 5 saved in memory 3.

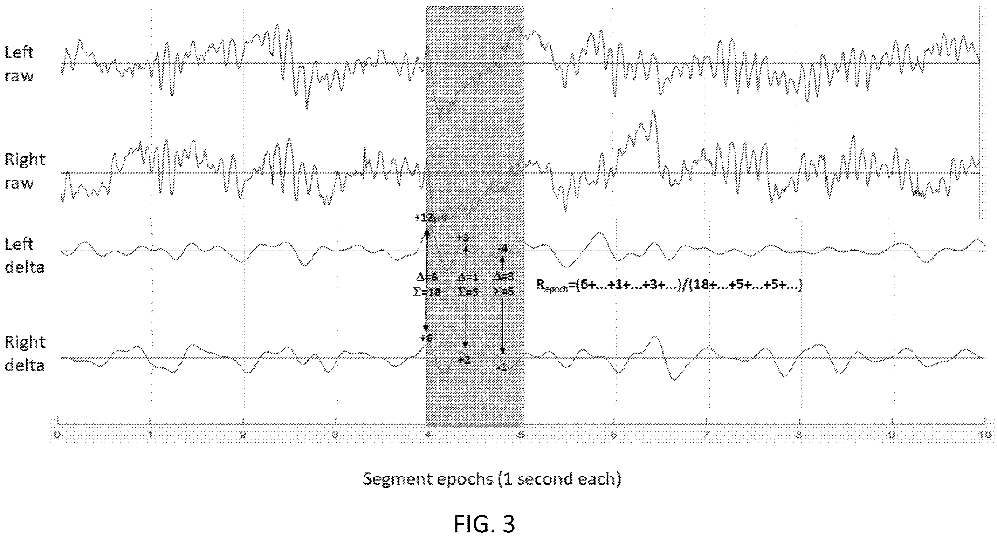

In step 210, a first EEG signal may be received from an electrode placed on the first (e.g., right) side of the head of a patient at a location allowing detecting an electrical activity of a first hemisphere of the brain. For example, computing device 10 (in Fig. 1B) may receive the first EEG signal form target electrode 30A (in Fig 1B). In step 220, a second EEG signal may be received from an electrode placed on the second (e.g., left) side of the head of the patient at a location allowing detecting an electrical activity of a second hemisphere of the brain. For example, computing device 10 may receive the second EEG signal form target electrode 30B. In some embodiments, target electrodes 30A and 30B may be placed as illustrated in Fig. 1A . An example, for such signals is given in the two upper recorded graphs of Fig. 3.

In step 230, a similarity index may be calculated for the electric activity between hemispheres of the brain based on the first and second signals. For example, computing device 10 may canulate the similarity index based on the amplitudes of the first signal and the second signal. Computing device 10 may calculate the similarity over time, by calculating at least one of: mean difference, median difference, standard deviation, percentile, variance and any combination thereof.

In some embodiments, calculating the similarity index may include calculating a correlation between the powers in the first signal and the second signal over time. In some embodiments, the correlation may be selected from, Pearson correlation and Spearman correlation and the like.

In some embodiments, the similarity index is calculated as differences in the power spectrum, between the first signal and the second signal, summarized for one of: specific frequencies, and/or a frequency band. In some embodiments, calculating the similarity index may include calculating, over time, at least of the following using the power spectrum of the first signal and the second signal: mean difference, median difference, standard deviation, percentile, variance and any combination thereof.

In some embodiments, calculating the similarity index may include calculating a correlation between the powers in the first signal and the second signal over the selected frequencies or frequency bands. In some embodiments, the correlation may be selected from, Pearson correlation and Spearman correlation, and the like.

In some embodiments, the similarity index may be calculated based on the degree of shifting in the distribution of frequency power spectrums between the first signal and the second signal. In some embodiments, the similarity index may be calculated based on the degree of shifting in the phases between the first signal and the second signal. Similarly, the similarity index may be calculated based on the degree of shifting in the distribution of power spectrums over time between the first signal and the second signal.

In a nonlimiting example, the similarity index may be a Lateral Interconnection Ratio (LIR) index. Computing device 10 may first filter the first and second signal to received filtered signals, for example, the two lower graphs in Fig. 3. The signals may be filtered to the delta bandpass (1-4Hz). As illustrated in Fig. 3 each 10 seconds segment is divided into 10 1-second epochs. Computing device 10 may then calculate subtraction (Δ) between the amplitudes and summation (Σ) of the amplitudes, for example, per sampling point. Computing device 10 may calculate the similarity index (LIR) as the ratio between the subtraction of the amplitudes and the summation of the amplitudes. In some embodiments, the ratio is calculated for a predetermined period of time, for example, every second, 0.5 seconds, 0.4 seconds and the like.

In some embodiments, the similarity index may be a combined similarity index, derived from various different indexes. The combined similarity index may be calculated using indexes calculated over time (i.e., in a time domain), calculated for various frequencies (i.e., in a frequency domain) and/or indexes calculated for various amplitudes (i.e., in a power domain).

In some embodiments, the first signal and the second signal may first be modified/filtered using any known method. For example, the methods may be filtering, ranging the data into several sub-ranges (starting from just two - e.g. greater and lesser from a threshold, or alternatively more than just two sub-ranges, according to any desired number of thresholds to define the ranges) , division to sub-periods, noise removal and the like. For example, filtering may be by any method selected from, low-pass, high-pass, or band-pass. In some embodiments, filtering may involve components analysis and evaluating of the occurrence of the component in the signal. The components may be of any type, including wavelets, etc.

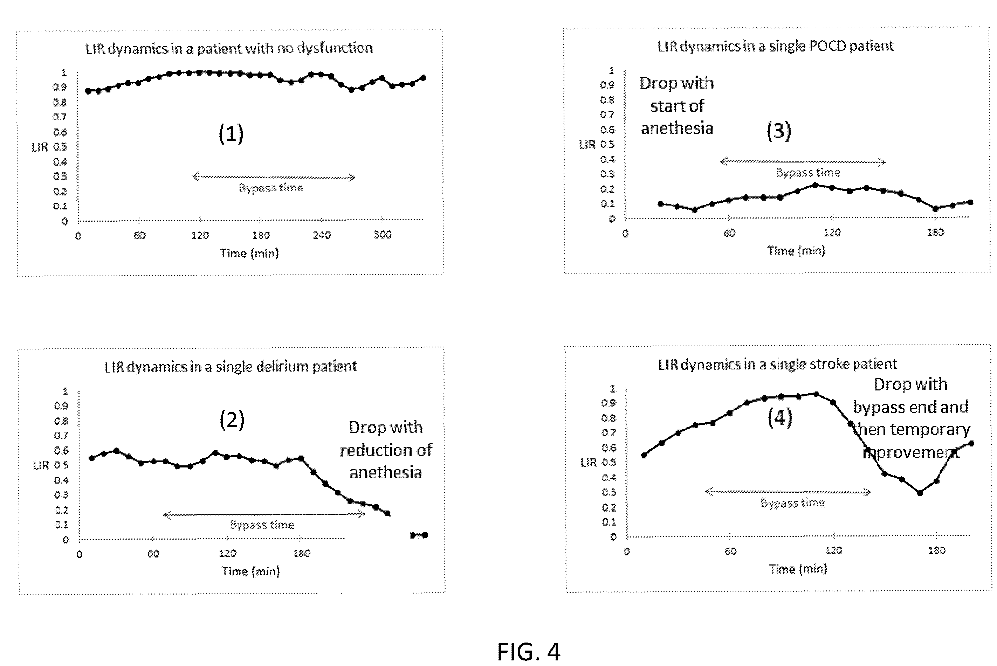

In step 240, a temporal change may be detected in the similarity index and may be determined if the similarity index is at least one of, (a) below a first threshold value; and (b) a time derivative of the similarity index is above a second threshold value. Nonlimiting examples for such temporal changes in LIR are illustrated in Fig. 4. As shown in the different graphs of Fig. 4 the temporal changes can be detected in the absolute value of the LIR (e.g., when comparing graph (1) to graph (3)) or the change/slope of the graphs, as shown in graphs (2) and (4) and discussed in detail hereinbelow with respect to Fig. 4.

In step 250, a medical-related input may be received. The medical-related input is at least one of, (a) an anesthetic profile during a medical procedure, and (b) input related to a medical event. In some embodiments, the anesthetic profile may be received from a user, via a user interface, or directly from the anesthetic-providing device. In some embodiments, receiving the medical-related input may include receiving a signal from at least one of, an external computing device and measurements received from at least one sensor (e.g., sensor 40). In some embodiments, the external computing device is selected from, a controller of a bypass machine, a controller of a medication provision machine, and the like. In some embodiments, the measurements (received from one or more sensors 40) are selected from: blood pressure, blood flow, temperature, saturation, glucose levels, and the like. In some embodiments, the sensors may be selected from: blood pressure sensors, thermometer, saturation sensor, US device, X-Ray device, and the like.

In some embodiments, receiving the medical-related input includes receiving an input from a user via a user interface. For example, medical personnel involved in the medical procedure may enter information related to the medical procedure (e.g., a surgery, an ICU, general hospitalization, emergency triage, etc.) during the medical procedure. In a nonlimiting example, the surgery may be cardiac surgery, a surgery in a sitting position, a vascular surgery and the like.

In some embodiments, information may include actions conducted by a medical professional during the medical procedure, events (e.g., bleeding) occurred during the medical procedure and the like. Some nonlimiting examples for such medical related input may include, during cardiac surgery, initiation of cardiopulmonary bypass, weaning from cardiopulmonary bypass, cannulation or de-cannulation of aorta, manipulation of the cardiac chambers and valves, cannulation of carotid artery for brain perfusion during total circulatory arrest; during surgeries (any type) in sitting position in which drop in brain perfusion or oxygenation to ranges which are considered undesired are likely, during cardiovascular interventions such as in carotid endarterectomy, catheterization, etc.

In some embodiments, a user device may include a graphical presentation (e.g., on a touchscreen) of a selection of various possible actions and events related to the medical procedure which may allow a simple selection of a required action/event during the medical procedure.

In step 260, determining the type of brain dysfunction based on the temporal change in the similarity index and the medical related input. Some nonlimiting examples for temporal behavior of LIR during cardiac surgery are given in the graphs of Fig. 4. Graph (1) shows the temporal LIR behavior of a normally functioning brain, graph (2) shows the temporal LIR behavior during delirium, graph (3) shows the temporal LIR behavior during post operative cognitive dysfunction (POCD) and graph (4) shows the temporal LIR behavior during a stroke. The graphs also indicating the time of, initiation of cardiopulmonary bypass and weaning from cardiopulmonary bypass.

In some embodiments, the brain dysfunction is POCD and determining the POCD is when the similarity index is below a POCD threshold value under certain anestethic profile. For example, when comparing the temporal LIR behavior of normal patient vs. a patient suffering from POCD, it clearly shows that the LIR values are much lower for a POCD patient than in a normal patient.

In some embodiments, the brain dysfunction is delirium that may occur during surgery (any surgery). Determining a delirium may be when: a time derivative of the similarity index is above a delirium threshold value, indicating a decrease in the similarity index with time, and the anesthetic profile shows temporal reduction in the amount of anesthetic. Risk for POCD is increased if the dissimilarity occurs following events, which may lead to focal cerebral blood flow dysregulation, such as anesthesia onset and change, relative hypotension or electrolyte imbalance (such as hypoglycemia), etc.

In some embodiments, delirium may occur at the end of cardiac surgery due to reduction in anesthesia level, relative hypoglycemia, overdose or under dose of anesthetic or analgesic medications, overdose of anticholinergic medications (such as atropine), pain, fever, infection, inflammation, etc. An example, showing a LIR temporal behavior during a delirium at the end of a cardiac surgery, is shown in graph (2) of Fig. 4, which shows a rapid drop in LIR following reduction in anesthetic.

In some embodiments, the brain dysfunction is delirium that may occur in an ICU. In the ICU, delirium risk is increased if the dissimilarity occurs when the patient is in the process of awakening from anesthesia, sedation, or any type of reduced consciousness.

In some embodiments, the brain dysfunction is stroke and determining the stroke is when the time derivative of the similarity index is above a first stroke threshold value and the input related to a medical event includes an input related to cardiac surgery. Stroke risk may be increased if the dissimilarity occurs following events which may cause traveling of emboli to the brain, e.g., during cardiac surgery. In some embodiments, the input related to cardiac surgery is selected from initiation of cardiopulmonary bypass and weaning from cardiopulmonary bypass received from a controller of a bypass machine. In some embodiments, the input related to cardiac surgery is selected from: cannulation or de-cannulation of the aorta, manipulation of the cardiac chambers and valves, and cannulation of carotid artery for brain perfusion during total circulatory arrest, received from a user via a user device. The temporal LIR behavior during stroke at cardiac surgery is given in graph (4) of Fig. 4. As shown the LIR behavior includes 3 changes in the time derivative of the LIR following events such as initiation of cardiopulmonary bypass, weaning from cardiopulmonary bypass, and reduction in anesthetics.

In some embodiments, stroke may occur during surgeries (any type) in a sitting position in which drop in brain perfusion or oxygenation to ranges which are considered undesired are likely. Therefore, the input related to the surgery may include inputs received from sensors, such as, sensors 40 discussed hereinabove.

As should be understood by one skilled in the art, LIR is given as an example for the similarity index. Other similarity indexes are expected to have similar behavior as the one demonstrated by LIR.

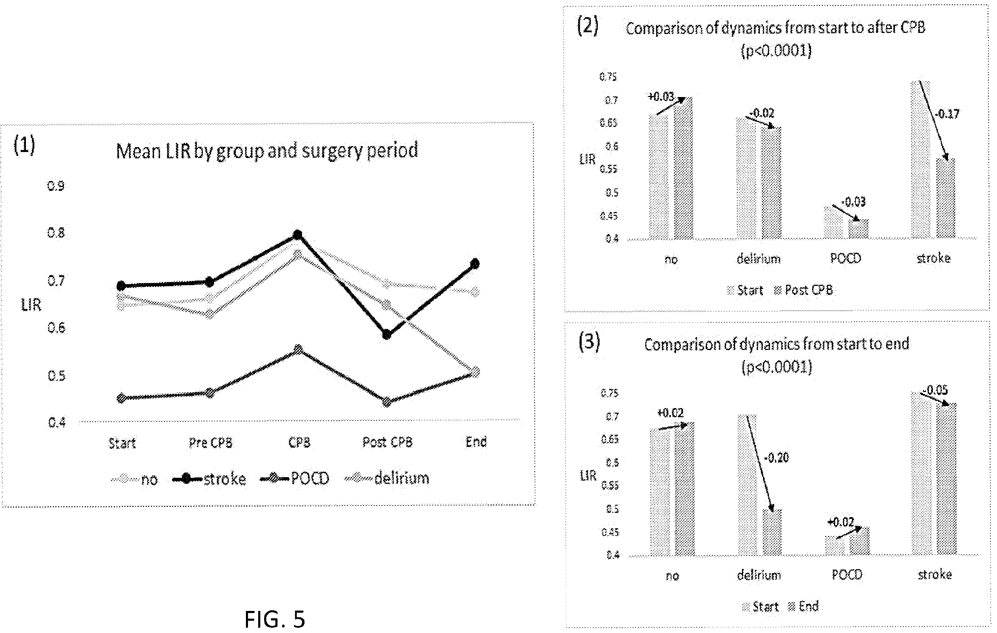

Reference is now made to Fig. 5 which shows graphs of accumulated LIR data gathered from groups of patients having several types of dysfunctions in comparison with patients having a normal brain function, according to some embodiments of the invention. The data in the sub-figures was taken from studies involving altogether more than a thousand patients who underwent cardiac and orthopedic surgeries. The prevalence of postoperative stroke was ~4%, of postoperative delirium ~6% and of POCD ~20%.

Sub-figure (1) shows the dynamics of each of the four patient groups (normal brain function, dysfunction, delirium, POCD, and stroke) among the major cardiac surgery periods (surgery start, pre bypass, on bypass, post bypass and surgery end). In the delirium group, it is possible to see the drop at surgery end. In the POCD group, low LIR values were detected from the onset of anesthesia at surgery start. In the stroke group, a drop in the LIR values was detected at the post bypass period, which was partly corrected at the surgery end. Sub-figures (2) and (3) present a comparison between the surgery start and post bypass period (sub-figure 2) and the surgery start and surgery end periods (sub-figure 3). As clearly shown in the graphs, the differences between the patient groups are significant with the ability to identify delirium, POCD, and stroke according to timing during surgery with specificity and sensitivity of over 0.7 (not shown).

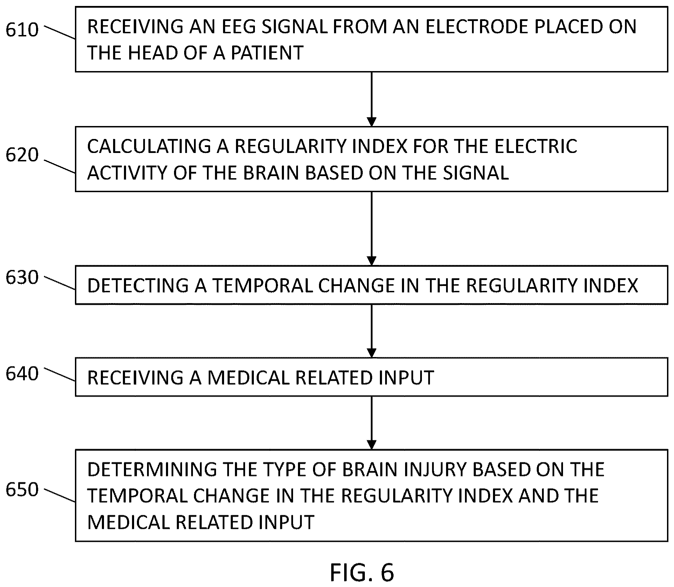

Reference is now made to Fig. 6 which is a flowchart of a method of determining a brain dysfunction, according to some embodiments of the invention. The method of Fig. 6 may be executed by any computing device, for example, computing device 10 based on a code 5 saved in memory 3.

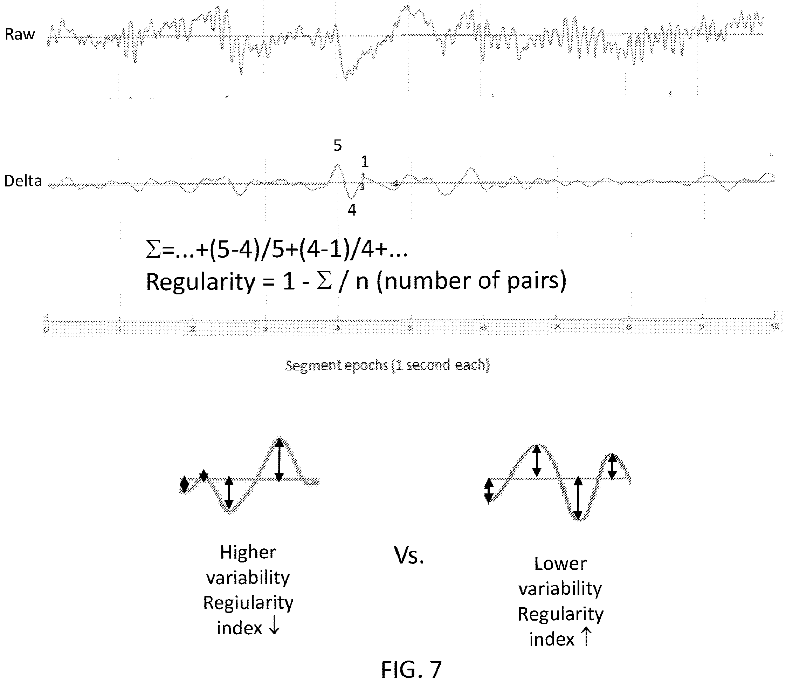

In step 610, an EEG signal may be received from an electrode placed on the head of a patient at a location allowing detecting an electrical activity of the brain. For example, computing device 10 of system 200 (in Fig. 1D) may receive the EEG signal from target electrode 30. For example, computing device 10 may receive the EEG signal form target electrode 30. In some embodiments, target electrode 30 may be placed as illustrated in Fig. 1C. An example, for such a signal is given in the upper recorded graph of Fig. 7.

In step 260, a regularity index may be calculated for the electric activity based on the signal. For example, computing device 10 may calculate the regularity index based on the amplitudes of the signal. Computing device 10 may calculate the regularity over time, by calculating at least one of: mean regularity, median regularity, standard deviation, percentile, variance, and any combination thereof.

In some embodiments, calculating the regularity index may include calculating a variability in the amplitude of the signal over time. In some embodiments, the variability may be computed on the differences between consecutive wave amplitudes. The variability may be computed with variance, standard deviation, mean, median, percent of values beyond and/or below and/or between thresholds, etc.

In some embodiments, the regularity index is calculated on the basis of the power spectrum of the signal, summarized for one of: specific frequencies, and/or a frequency band. In some embodiments, calculating the regularity index may include calculating, over time, at least one of the following using the power spectrum of the signal: mean regularity, median regularity, standard deviation, percentile, variance and any combination thereof.

In some embodiments, calculating the regularity index may include calculating a variability in the power of the signal over the selected frequencies or frequency bands. In some embodiments, the variability may be computed on the differences between consecutive wave amplitudes. The variability may be computed with variance, standard deviation, mean, median, percent of values beyond and/or below and/or between thresholds, etc.

In a nonlimiting example, the regularity index may be a Brain Injury Index (BII). Computing device 10 may first filter the signal to a received filtered signal, for example, the lower graph in Fig. 7. The signal may be filtered to the delta band pass (1-4Hz). As illustrated in Fig. 7, each 10 seconds segment is divided into 10 1-second epochs. Computing device 10 may then calculate delta (Δ) between the consecutive wave absolute amplitudes, and the ratio of each delta to the highest amplitude of the two waves involved in each pair, and then summarize (Σ) these ratios and divide them by the number of the pairs.

In some embodiments, the regularity index may be a combined regularity index, derived from various different regularity index computations, as suggested above. The combined regularity index may be calculated using indexes calculated over time (i.e., in a time domain) and/or calculated for various frequencies (i.e., in a frequency domain).

In some embodiments, the signal may first be modified/filtered using any known method. For example, the methods may be filtering ranging, division to sub-periods, noise removal and the like. For example, filtering may be by any method selected from, low-pass, high-pass, or band-pass. In some embodiments, filtering may involve components analysis and evaluating of the occurrence of the component in the signal. The components may be of any type, including wavelets, etc.

In step 630, a temporal change may be detected in the regularity index and may be determined if the regularity index is at least one of, (a) below a first threshold value; and (b) a time derivative of the regularity index is above a second threshold value. Nonlimiting examples for such temporal changes in BII are illustrated in Fig. 4 and 5. As shown in the different graphs of Fig. 4 the temporal changes can be detected in the absolute value of the BII (e.g., when comparing graph (1) to graph (3)) or the change/slope of the graphs, when comparing graphs (2) and (3).

In step 240, a medical related input may be received. The medical related input is at least one of, (a) a medical history related to the patient, (b) an input related to the surgery type and complexity, (c) an anesthetic profile during a medical procedure, and (d) input related to a medical event. In some embodiments, the anesthetic profile may be received from a user, via a user interface, or directly from the anesthetic providing device. In some embodiments, receiving the medical related input may include receiving a signal from at least one of, an external computing device and measurements received form at least one sensor (e.g., sensor 40). In some embodiments, the external computing device is selected from, a controller of a bypass machine, a controller of a medication provision machine and the like. In some embodiments, the measurements (received from one or more sensors 40) are selected from: blood pressure, blood flow, temperature, saturation, glucose levels, movements and the like. In some embodiments, the sensors may be selected from: blood pressure sensors, thermometer, saturation sensor, US device, X-Ray device, movement sensors and the like.

In some embodiments, receiving the medical related input includes receiving an input from a user via a user interface. For example, a medical personnel involved in the medical procedure may enter information related to the medical procedure (e.g., a surgery, an ICU, a PICU or NICU, general hospitalization, emergency triage, etc.) during the medical procedure. In a nonlimiting example, the surgery may be a cardiac surgery, a surgery in a sitting position, a vascular surgery and the like.

In some embodiments, information may include actions conducted by a medical professional during the medical procedure, events (e.g., bleeding) occurred during the medical procedure and the like. Some nonlimiting examples for such medical related input may include, during cardiac surgery, weaning to and from cardiopulmonary bypass, cannulation or de-cannulation of aorta, manipulation of the cardiac chambers and valves, cannulation of carotid artery for brain perfusion during total circulatory arrest; during surgeries (any type) in sitting position in which drop in brain perfusion or oxygenation to ranges which are considered undesired are likely, during cardiovascular interventions such as in carotid endarterectomy, catheterization, etc.

In some embodiments, a user device may include a graphical presentation (e.g., on a touchscreen) of a selection of various possible actions and events related to the medical procedure which may allow a simple selection of a required action/event during the medical procedure.

In some embodiments, receiving input related to the patient's medical history may include at least one of: an age of the patient, a baseline cognitive profile and cardiovascular risk assessment. For example, previous stroke or transient ischemic attack (TIA), old myocardial infraction (MI), peripheral vascular disease (PVD), non insulin or insulin dependent diabetes mellitus (NIDDM/ IDDM), status of pre-operative cognitive dysfunction (such as Alzheimer disease) and previous delirium states, sleep apnea, obesity and functional capacity and the like.

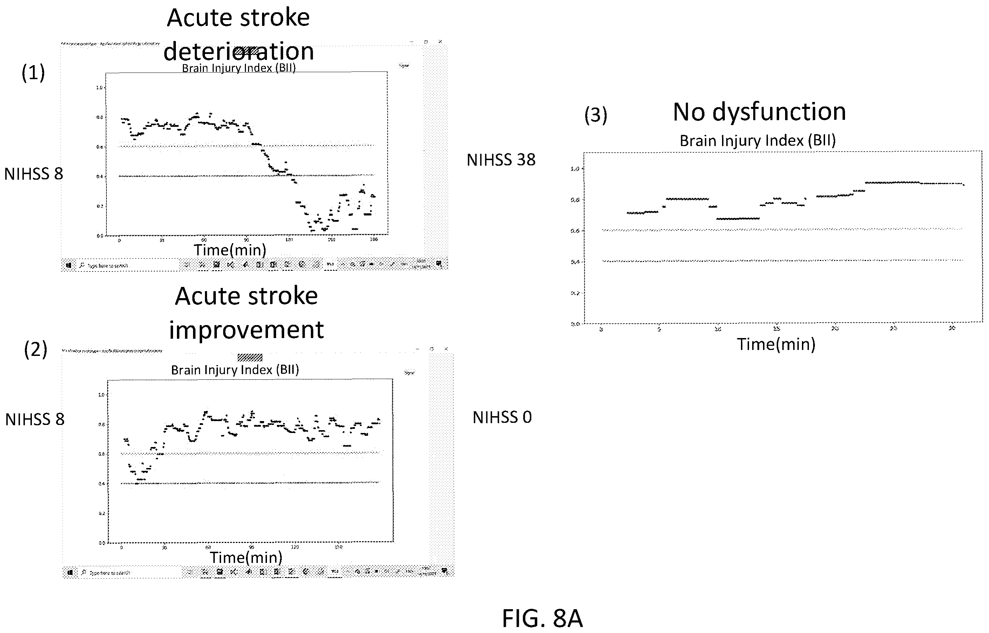

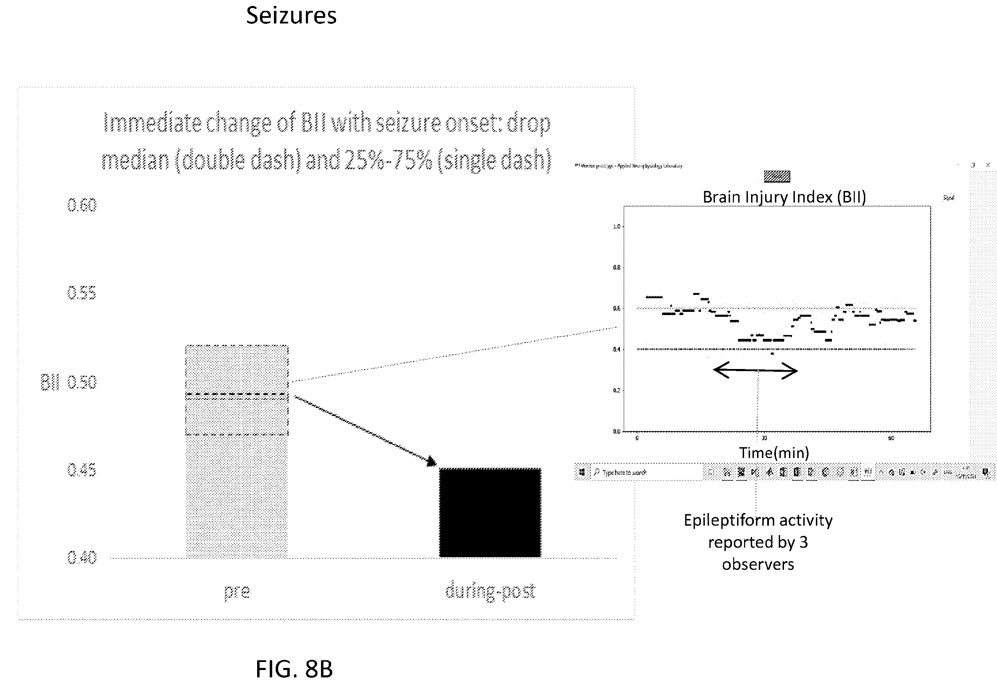

In step 650, the type of brain dysfunction may be determined based on the temporal change in the regularity index and the medical related input. Some nonlimiting examples for temporal behavior of BII of patients are given in the graphs of Fig. 8. Graph (1) shows the temporal BII behavior of a patient with stroke during unsuccessful catheterization, graph (2) shows the temporal BII behavior of a stroke patient during successful catheterization, graph (3) shows the temporal BII behavior during surgery without neurological complications. Graph (4a) shows the temporal BII behavior during a seizure. Graph (4b) shows the population summary of BII with seizure onset.

In some embodiments, the brain dysfunction is POCD and determining the POCD is when the regularity index is below a POCD threshold value under certain anestethic profile. For example, when comparing the temporal BII behavior of normal patient vs. a patient suffering from POCD, it clearly shows that the BII values are much lower for a POCD patient than in a normal patient.

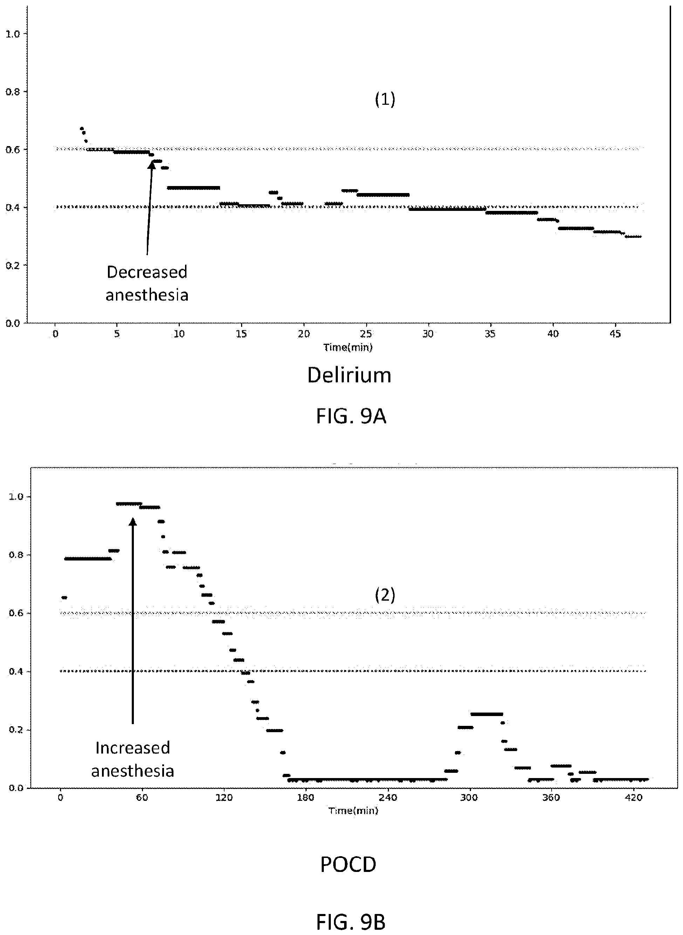

In some embodiments, the brain dysfunction is delirium that may occur during surgery (any surgery). Determining a delirium may be when: a time derivative of the regularity index is above a delirium threshold value, indicating a decrease in the regularity index with time, and the anesthetic profile shows temporal reduction in the amount of anesthetic/analgesic. Risk for POCD is increased if the irregularity occurs following events, which may lead to focal cerebral blood flow dysregulation, such as anesthesia onset and change, relative hypotension or electrolyte imbalance (such as hypoglycemia), etc. An example, showing a BII temporal behavior with POCD in the graph of Fig. 9A, which shows a drop in BII following increase in anesthetic.

In some embodiments, delirium may occur at the end of cardiac surgery due to reduction in anesthesia/analgesia level, relative hypoglycemia, overdose or under dose of anesthetic or analgesic medications, overdose of anticholinergic medications (such as atropine), pain, fever, infection, inflammation, etc. An example, showing a BII temporal behavior with delirium in the graph of Fig. 9B, which shows a drop in BII following reduction in analgesic.

In some embodiments, the brain dysfunction is delirium that may occur in an ICU. In the ICU, delirium risk is increased if the irregularity occurs when the patient is in the process of awakening from anesthesia, sedation, or any type of reduced consciousness.

In some embodiments, the brain dysfunction is stroke and determining the stroke is when the time derivative of the regularity index is above a first stroke threshold value and the input related to a medical event includes an input related to a cardiac surgery. Stroke risk may be increased if the irregularity occurs following events which may cause travelling of emboli to the brain, e.g., during cardiac surgery. In some embodiments, the input related to a cardiac surgery is selected from: weaning from cardiopulmonary bypass received from a controller of a bypass machine. In some embodiments, the input related to a cardiac surgery is selected from: cannulation or de-cannulation of aorta, manipulation of the cardiac chambers and valves and cannulation of carotid artery for brain perfusion during total circulatory arrest, received from a user via a user device.

In some embodiments, stroke may occur during surgeries (any type) in sitting position in which drop in brain perfusion or oxygenation to ranges which are considered undesired are likely. Therefore, the input related to the surgery may include inputs received from sensors, such as, sensors 40 discussed hereinabove.

As should be understood by one skilled in the art, BII is given as an example for the regularity index. Other regularity indexes are expected to have similar behavior as the one demonstrated by BII.

In some embodiments, determining the type of brain dysfunction may allow the medical personnel to give proper treatment in time to the patient. In some embodiments, the diagnosis of brain dysfunction a set of recommendations may be derived. In some embodiments, recommendations may be implemented manually by the medical personnel. Additionally and alternatively, recommendation and/or action may be carried out , by way of controlling various interventional devices, for example, the anesthetic provision device. Some nonlimiting examples for such recommendations and actions are listed below.

In some embodiments, when a high risk for POCD is determined one of the following actions may be considered:
- Reducing/not increasing level of anesthesia, if provided in continuous drip (decrease dose) or inhalation (decrease MAC), or if doses are gradually increased to begin with.
- Improve head position.
- Titrate level of brain vasoconstriction pharmacologically or by titrating blood CO₂ level.
- Assess essential blood loss (EBL) during surgery and the need for blood transfusion which could improve level of brain oxygenation by increasing level of oxygen carrying capacity.
- Assess oxygen saturation and improve oxygenation by titrating the ventilation (increase FIO2, increase PEEP, change mode of ventilation).
- Improve level of brain oxygenation by pharmacological or hemodynamic titration of blood pressure and/or cardiac output and/or level of hemoglobin.

In some embodiments, when a high risk for stroke is determined it is possible to:
- Alert the relevant neurological team (could also be done automatically) for immediate evaluation and treatment as needed (even immediately after an operation, or even during an operation).
- Improve level of brain oxygenation by increasing level of blood oxygen saturation.
- Improve level of brain oxygenation by pharmacological or hemodynamic titration of blood pressure and/or cardiac output and/or level of hemoglobin.

In some embodiments, when a high risk for delirium is determined high it is possible to:
- Control environmental conditions to enable a proper circadian circle - such as proper illumination, noise reduction and adding a clock.
- Maintain proper mobility to reduce risk for delirium and deterioration.
- Remove as soon as possible any unnecessary lines such as urinary catheter, chest tubes, IV lines, etc.
- Treat with hypnotic drugs if the patient suffers from insomnia.
- Treat with analgesics to reduce a suspected pain.
- Treat with antipyretics in case of fever.
- Treat with relevant antibiotics or with relevant anti-inflammatory drugs, in case infection or otherwise inflammation, are considered respectively.
- Treat with anxiolytics or antipsychotics if the patient suffers from anxiety or psychosis respectively.

Avoid long-acting benzodiazepines, meperidine and anti-cholinergic drugs.

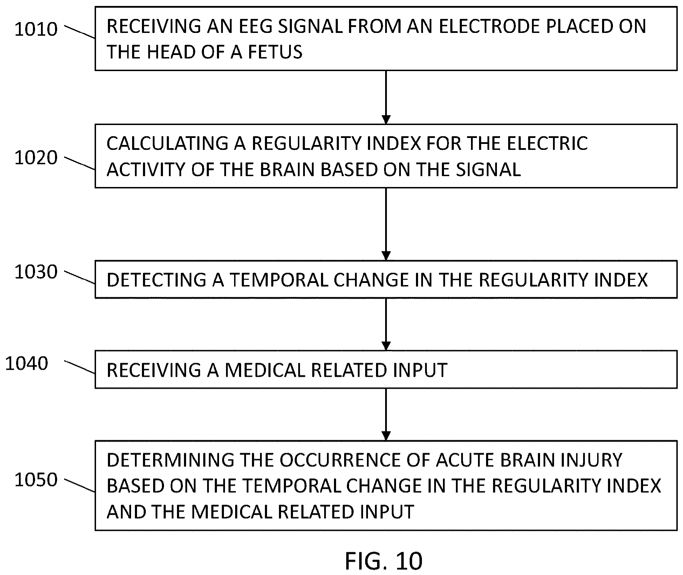

Reference is now made to Fig. 10, which is a flowchart of a method of determining an acute brain dysfunction according to some embodiments of the invention. The method of Fig. 10 may be executed by any computing device, for example, computing device 10 based on a code 5 saved in memory 3.

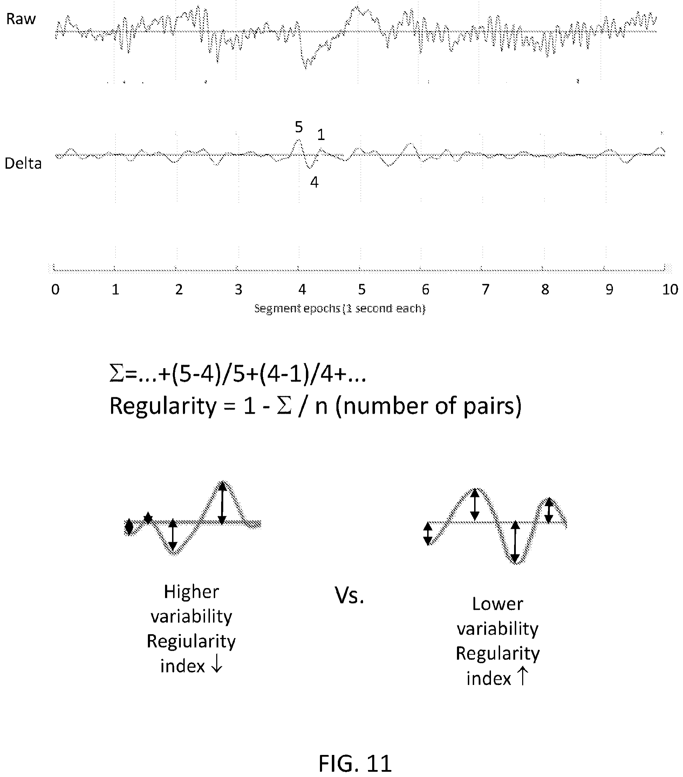

In step 1010, an EEG signal may be received from an electrode placed on the head of a fetus at a location allowing detecting an electrical activity of the brain. For example, computing device 10 may receive the EEG signal form the target electrode 30. In some embodiments, the target electrode 30 may be placed as illustrated in Fig. 1E. An example, for such signals is given in the upper recorded graph of Fig. 11. In some embodiments, the EEG signal may be received directly from an EEG electrode or may be extracted from cardiotocography (CTG) data, electrocardiogram (ECG) and the like.

In step 1020, a regularity index may be calculated for the electric activity based on the signal. For example, computing device 10 may calculate the regularity index based on the amplitudes of the signal. Computing device 10 may calculate the regularity over time, by calculating at least one of: mean regularity, median regularity, standard deviation, percentile, variance and any combination thereof.

In some embodiments, calculating the regularity index may include calculating a variability in the amplitude of the signal over time. In some embodiments, the variability may be computed on the differences between consecutive wave amplitudes. The variability may be computed with variance, standard deviation, mean, median, percent of values beyond and/or below and/or between thresholds, etc.

In some embodiments, the regularity index is calculated on the basis of the power spectrum of the signal, summarized for one of: specific frequencies, and/or a frequency band. In some embodiments, calculating the regularity index may include calculating, over time, at least one of the following using the power spectrum of the signal: mean regularity, median regularity, standard deviation, percentile, variance and any combination thereof.

In some embodiments, calculating the regularity index may include calculating a variability in the power of the signal over the selected frequencies or frequency bands. In some embodiments, the variability may be computed on the differences between consecutive wave amplitudes. The variability may be computed with variance, standard deviation, mean, median, percent of values beyond and/or below and/or between thresholds, etc.

In a nonlimiting example, the regularity index may be a Brain Injury Index (BII). Computing device 10 may first filter the signal to a received filtered signal, for example, the lower graph in Fig. 11. The signal may be filtered to the delta band pass (1-4Hz). As illustrated in Fig. 10, each 10 seconds segment is divided into 10 1-second epochs. Computing device 10 may then calculate delta (Δ) between the consecutive wave absolute amplitudes, and the ratio of each delta to the highest amplitude of the two waves involved in each pair, and then summarize (Σ) these ratios and divide them by the number of the pairs.

In some embodiments, the regularity index may be a combined regularity index, derived from various different regularity index computations, as suggested above. The combined regularity index may be calculated using indexes calculated over time (i.e., in a time domain) and/or calculated for various frequencies (i.e., in a frequency domain).

In some embodiments, the signal may first be modified/filtered using any known method. For example, the methods may be filtering ranging, division to sub-periods, noise removal and the like. For example, filtering may be by any method selected from, low-pass, high-pass, or band-pass. In some embodiments, filtering may involve components analysis and evaluating of the occurrence of the component in the signal. The components may be of any type, including wavelets, etc.

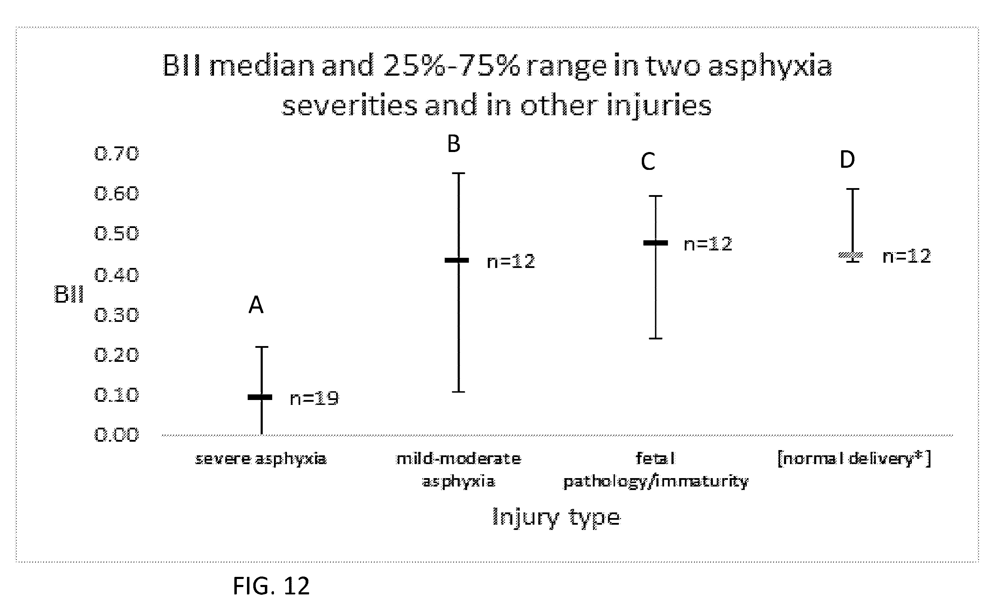

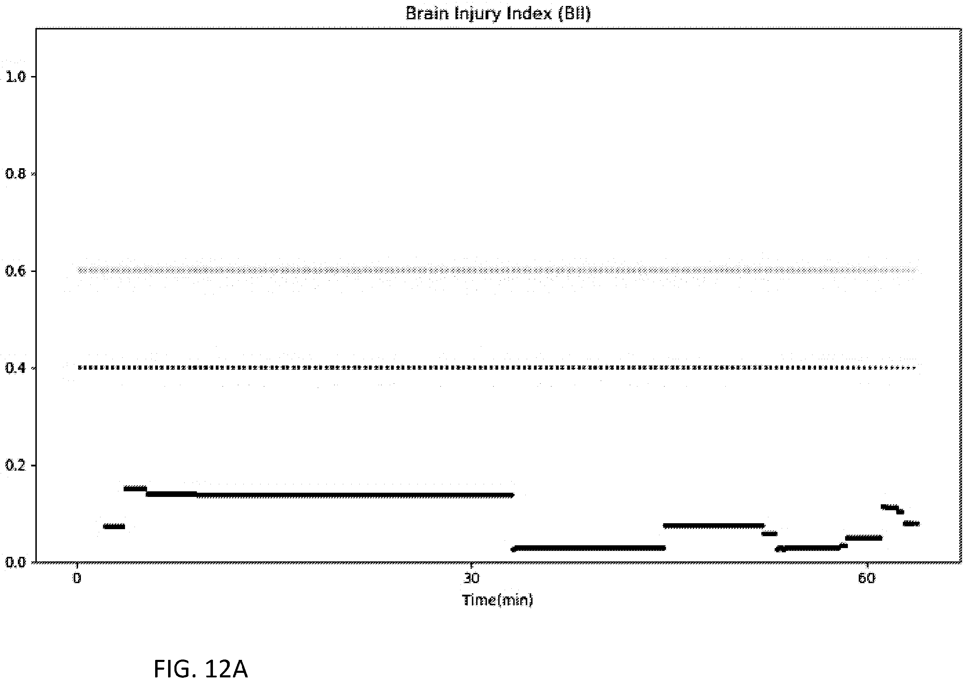

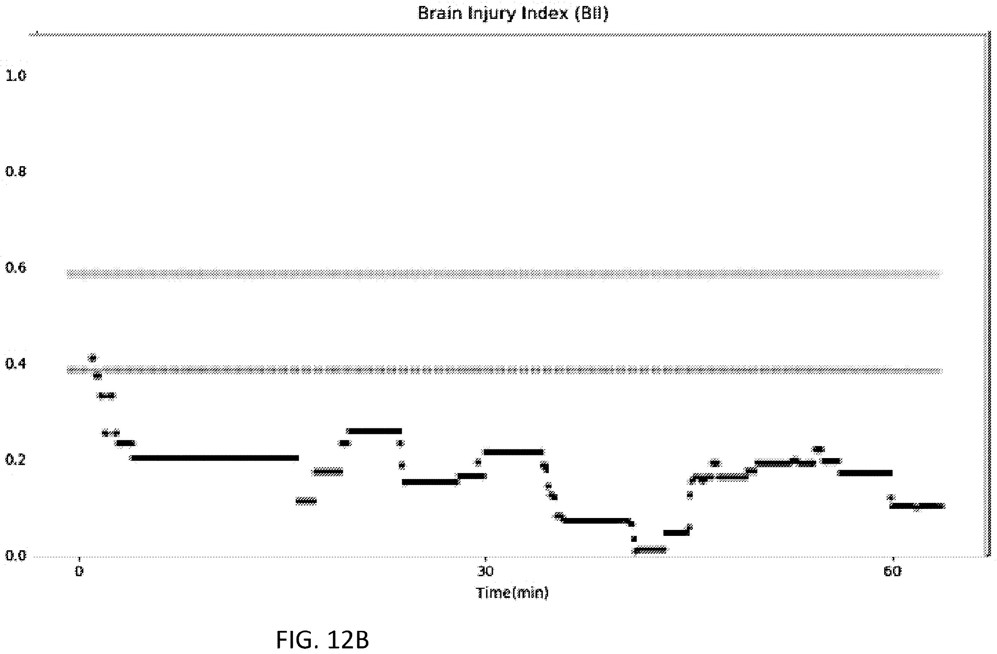

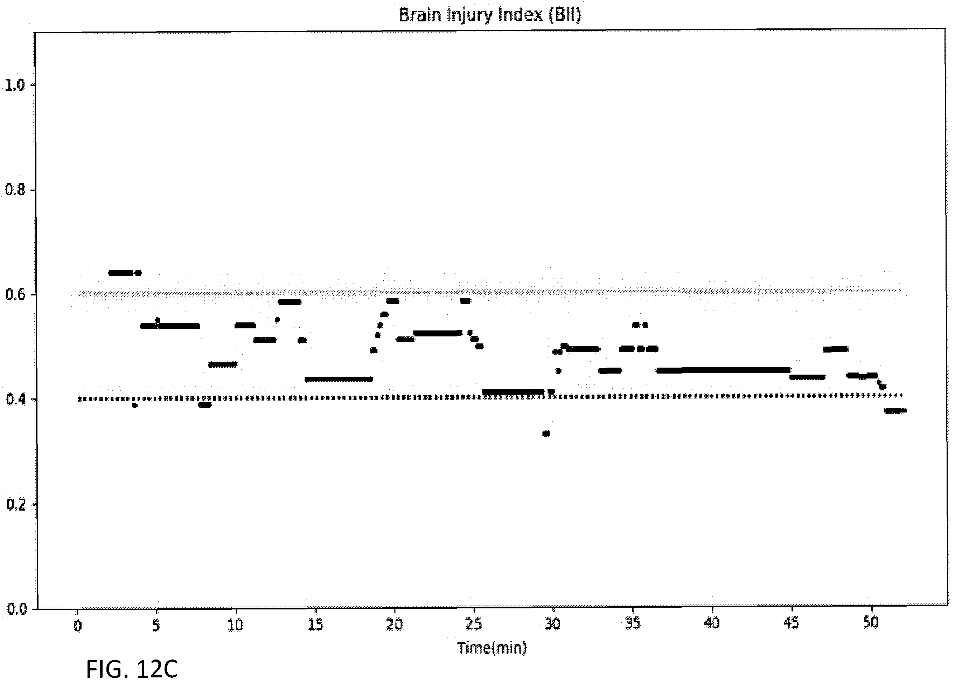

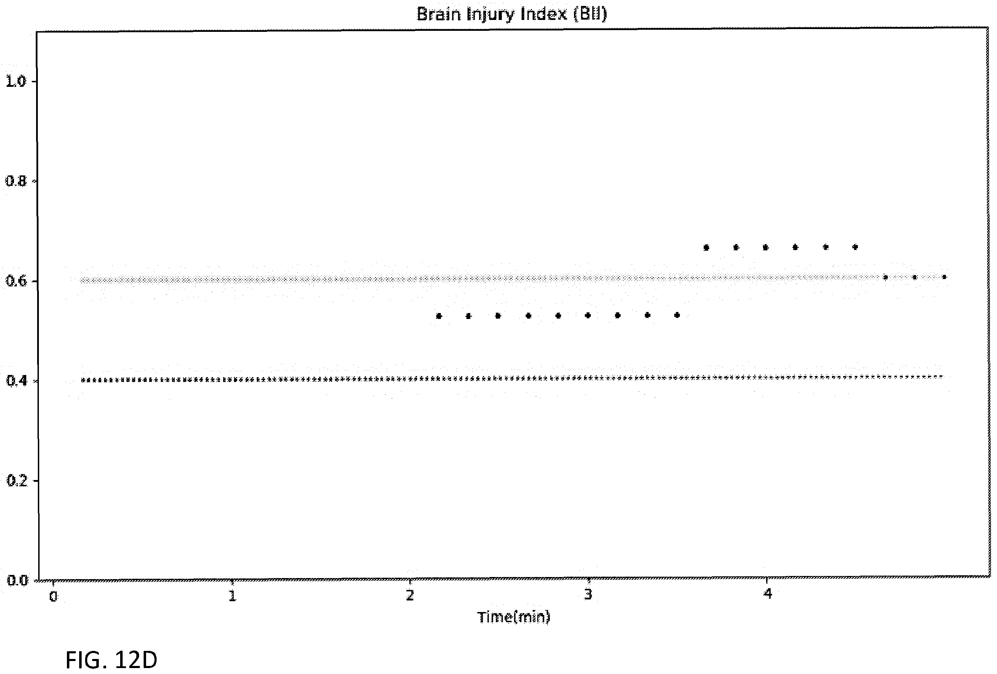

In step 1030, a temporal change may be detected in the regularity index and may be determined if the regularity index is at least one of, (a) below a first threshold value; and (b) a time derivative of the regularity index is above a second threshold value. Nonlimiting examples for such temporal changes in BII are illustrated in Figs. 12-12D. As shown in the graphs 12A, 12B, 12C and 12D which shows temporal changes detected in the absolute value of the BII and are related to the severity of acute intra-partum asphyxia and brain injury. The Graph of Fig. 12A corresponds to severe asphyxia as shown in point A in the graph of Fig. 12. Similarly, the graph of Fig. 12B corresponds to mild-moderate asphyxia (point B), the graph of Fig. 12C corresponds to fetal pathology/immaturity (point C) and the graph of Fig. 12D corresponds to normal delivery (point D). As clearly shown in the graphs, during normal delivery the BII indexes are substantially higher while any one of the graphs 12A-12C shows lower values that is typical for each specific type of acute brain dysfunction. This is summarized in the main figure 12 at the group level.

In step 1040, a medical related input may be received. The medical related input could be, for example: (a) input regarding aberrant fetus ECG activity, e.g., late decelerations or reduced variability, and (b) input related to contractions. In some embodiments, the medical related input may be received from a user, via a user interface, or directly from the relevant devices. In some embodiments, receiving the medical related input may include receiving a signal from at least one of, an external computing device and measurements received form at least one sensor (e.g., sensor 40). In some embodiments, the external computing device is selected from, a controller of an ECG machine, a controller of a tocodynamometer and the like. In some embodiments, the sensors may be selected from: US device and the like.

In some embodiments, receiving the medical related input includes receiving an input from a user via a user interface. For example, medical personnel involved in the medical procedure may enter information related to the medical procedure (such as the labor stage and progression) and the medical condition of the mother, before and during the delivery.

In some embodiments, information may include actions conducted by a medical professional during the medical procedure, events (e.g., contractions) occurred during the medical procedure and the like.

In some embodiments, a user device may include a graphical presentation (e.g., on a touchscreen) of a selection of various possible actions and events related to the medical procedure which may allow a simple selection of a required action/event during the medical procedure.

In step 1050, determining the occurrence of an acute brain dysfunction based on the temporal change in the regularity index. In some embodiments, the acute brain dysfunction may also be determined based on the medical related input. Some nonlimiting examples for temporal behavior of BII of patients are given in the graphs of Figs. 12-12D. The graph of Fig 12 shows the differences in BII according to severity of asphyxia and related brain injury, and in comparison with congenital or pregnancy related pathologies and normal delivery. The graphs in Figs. 12A-12D present the BII data from representative newborns and fetuses in each category of severe asphyxia, mild-moderate asphyxia, congenital anomalies and prematurity and normal delivery.

As should be understood by one skilled in the art, BII is given as an example for the regularity index. Other regularity indexes are expected to have similar behavior as the one demonstrated by BII.

In some embodiments, determining the occurrence of an acute brain dysfunction may allow the medical personnel to give a proper treatment in time to the patient. In some embodiments, with the diagnosis of brain dysfunction a set of recommendations may be derived. In some embodiments, recommendations may be implemented manually by the medical personnel. Recommendations could involve interventions to expedite delivery according to known procedures - for example immediate Cesarean section, Vacuum delivery, etc.

Unless explicitly stated, the method embodiments described herein are not constrained to a particular order or sequence. Furthermore, all formulas described herein are intended as examples only and other or different formulas may be used. Additionally, some of the described method embodiments or elements thereof may occur or be performed at the same point in time.

While certain features of the invention have been illustrated and described herein, many modifications, substitutions, changes, and equivalents may occur to those skilled in the art. It is, therefore, to be understood that the appended claims are intended to cover all such modifications and changes as fall within the true spirit of the invention.

Various embodiments have been presented. Each of these embodiments may of course include features from other embodiments presented, and embodiments not specifically described may include various features described herein.

## Claims

1. A method of determining a brain dysfunction, comprising:
receiving an electroencephalogram (EEG) signal from an electrode placed on a patient' head at a location allowing detecting electrical activity of the brain;
calculating regularity index for the electric activity of the brain based on the EEG signal; and
detecting a regularity change in the regularity index by determining if the regularity index is at least one of:
(c) below a first threshold value; and
(d) a time derivative of the regularity index is above a second threshold value;
receiving a medical related input, wherein the medical related input is at least one of:
(iv) input related to the patient's medical history;
(v) an anesthetic profile during a medical procedure; and
(vi)an input related to a medical event; and
determining a type of brain dysfunction based on the temporal change in the regularity index and the medical related input.

2. The method of claim 1, wherein the receiving input related to the patient related to the patient's medical history comprising at least one of: an age of the patient, a baseline cognitive profile and cardiovascular risk assessment.

3. The method according to any one of claims 1 to 2, wherein receiving the medical related input includes receiving a signal from at least one of: an external computing device and measurements received form at least one sensor.

4. The method of claim 3, wherein the input related to the medical event measurements is selected from: blood pressure, blood flow, temperature, saturation, glucose levels, other hemodynamic parameters, such as, pre-operative demographic and/or clinical data, ECG, central venous pressure and arterial invasive pressure, patient movements, target control infusion pumps of anesthetic medications (TCI) and patient movements monitoring.

5. The method of claim 3, wherein the external computing device is selected from a data storage with an electronic medical record, a controller of a bypass machine, a controller of a medication provision machine, a motion monitoring device, and a controller of the anesthetic machine.

6. The method according to any one of claims 1 to 5, wherein the input related to the medical event is received from one of: electronic medical record, blood pressure sensors, thermometer, camera, saturation sensor, US device, X-Ray device, end-tidal CO₂ (EtCO2) detector, motion detector, and transcranial Doppler.

7. The method according to any one of claims 1 to 6, wherein receiving the input related to the medical event includes receiving an input from a user via a user interface.

8. The method according to any one of claims 1 to 7, wherein the brain dysfunction is perioperative neurocognitive dysfunction (PND).

9. The method of claim 8, wherein the PND includes preoperatively diagnosed cognitive decline, postoperative delirium, delayed neurocognitive recovery, and postoperative cognitive dysfunction (POCD).

10. The method of claim 9, wherein determining a POCD is when the regularity index is below a POCD threshold value under certain anesthetic profile.

11. The method according to any one of claims 1 to 10, wherein the brain dysfunction is delirium and wherein determining delirium is when: a time derivative of the regularity index is above a delirium threshold value, indicating a decrease in the regularity index with time, and the anesthetic profile shows temporal reduction in the amount of anesthetic.

12. The method according to any one of claims 1 to 10, wherein the brain dysfunction is a stroke and wherein determining stroke is when the time derivative of the regularity index is above a first stroke threshold value and the input related to a medical event includes an input related to a surgery.

13. The method according to any one of claims 1 to 12, wherein the brain dysfunction is a seizure and wherein determining seizure is when: a time derivative of the regularity index is above a seizure threshold value, indicating a decrease in the regularity index with time, followed by a time derivative of the regularity index above another seizure threshold value.

14. The method according to any one of claims 1 to 13, wherein the regularity index is calculated based on at least one of:
the amplitude of the signal;
the change between consecutive wave amplitudes. In some embodiments, the index is calculated for a predetermined period of time;
the power spectrum, of the signal, summarized for one of: specific frequencies, and a frequency band; and
a degree of shifting in power spectrums distribution over specific frequencies or a band of frequencies in the signal.

15. The method according to any one of claims 1 to 14, wherein calculating the regularity index comprises at least one of:
calculating, over time, at least one of the following using the amplitude of the signal: mean variability, median variability, standard deviation, percentile, variance and any combination thereof;
calculating, over time, at least one of the following using the power spectrum of the signal: mean regularity, median regularity, standard deviation, percentile, variance and any combination thereof;
calculating powers in the signal over the selected frequencies or the frequency bands, which associate with decreased or increased regularity; and calculating the powers in the signal over the selected periods of time.
